# EUROPEAN PATENT SPECIFICATION

(11) **EP 4 341 264 B1**
(45) Date of publication and mention of the grant of the patent: **02.04.2025**
(21) Application number: 22726190.6
(22) Date of filing: 18.05.2022
(51) Int. Cl.: C07D 487/04, A61P 33/14, A61K 31/519

(54) **PYRROLOPIRIMIDINE DERIVATIVES AND THEIR USE IN THE TREATMENT OF LEISHMANIASIS**
PYRROLOPIRIMIDINDERIVATE UND IHRE VERWENDUNG BEI DER BEHANDLUNG VON LEISHMANIOSE
DÉRIVÉS DE PYRROLOPIRIMIDINE ET LEUR UTILISATION DANS LE TRAITEMENT DE LEISHMANIOSE

(30) Priority: 20.05.2021 EP 21175055
(43) Date of publication of application: 27.03.2024
(73) Proprietor: Drugs for Neglected Diseases Initiative, 1202 Geneva (CH)
(72) Inventor: BREESE, Karen, Applecross, WA 6153 (AU); CHATELAIN, Eric, 4600 Olten (CH); HEPPELL, Jacob, Kensington, WA 6151 (AU); KEENAN, Martine, Karrinyup, WA 6018 (AU); VON GELDERN, Thomas, Richmond, Illinois 6007 (US)
(74) Representative: P&TS SA (AG, Ltd.)
(86) International application number: PCT/IB2022/054592
(87) International publication number: WO 2022/243877

(56) References cited:
- IMANOL PENA ET AL: "New Compound Sets Identified from High Throughput Phenotypic Screening Against Three Kinetoplastid Parasites: An Open Resource", SCIENTIFIC REPORTS, vol. 5, 5 March 2015 (2015-03-05), pages 8771, XP055211983, DOI: 10.1038/srep08771
- PENA: "New Compound Sets Identified from High Throughput Phenotypic Screening Against Three Kinetoplastid Parasites: An Open Resource - Supplementary Table 2", SCI.REP., 5 March 2015 (2015-03-05), XP055848764, Retrieved from the Internet <URL:https://static-content.springer.com/esm/art:10.1038/srep08771/MediaObjects/41598_2015_BFsrep08771_MOESM2_ESM.xlsx> [retrieved on 20211007]
- RAZZAGHI-ASL NIMA ET AL: "Insights into the current status of privileged-heterocycles as antileishmanial agents", MOLECULAR DIVERSITY, ESCOM SCIENCE PUBLISHERS, LEIDEN, NL, vol. 24, no. 2, 26 April 2019 (2019-04-26), pages 525 - 569, XP037152601, ISSN: 1381-1991, [retrieved on 20190426], DOI: 10.1007/S11030-019-09953-4
- KAPIL SWATI ET AL: "An update on small molecule strategies targeting leishmaniasis", EUROPEAN JOURNAL OF MEDICINAL CHEMISTRY, ELSEVIER, AMSTERDAM, NL, vol. 157, 6 August 2018 (2018-08-06), pages 339 - 367, XP085491741, ISSN: 0223-5234, DOI: 10.1016/J.EJMECH.2018.08.012

## Description

### Field of Invention

This invention relates to the use of pyrrolopyrimidine derivatives, and in particular pyrrolopyrimidine amine derivatives as anti-Leishmanial agents. Certain novel pyrrolopyrimidine derivatives are also disclosed together with processes for their preparation, intermediates thereto, pharmaceutical compositions comprising them, and their use in therapy.

### Background of the Invention

Leishmaniasis is a neglected tropical disease (NTD) that threatens an estimated one billion people worldwide and is endemic in 98 countries mainly in East Africa, Latin America, and South Asia. Leishmaniasis is a complex vector-borne disease, transmitted by more than 20 species of the protozoan genus *Leishmania,* and associated with clinical manifestations ranging from relatively benign localized skin ulcers to a systemic disease-causing severe damages to visceral organs that is fatal if left untreated. The different forms of the disease are categorized as visceral, cutaneous, mucocutaneous, and post-kala-azar dermal leishmaniasis (PKDL). Leishmaniasis breaks out in "foci", concentrated geographic areas, and is transmitted by the bite of a sand fly. The spread of the disease is impacted by environmental changes such as deforestation, building of dams, irrigation schemes, and urbanization. An estimated 700,000 to 1 million new cases of the disease in its various forms occur annually, with an estimated number of 20,000 to 30,000 associated deaths. The fatality rate of Visceral Leishmaniasis (VL), also called Kala-Azar, is over 95% if left untreated.

Visceral Leishmaniasis is predominantly caused by the protozoan parasites *L. donovani* and *L. infantum,* with a distribution in Asia, East Africa, Latin America, and the Mediterranean region. In patients who develop symptoms, presentation is insidious with development of splenomegaly, irregular fevers, anaemia, pancytopenia, weight loss and weakness occurring progressively over a period of weeks or even months.

Cutaneous Leishmaniasis (CL) is endemic in all tropical and subtropical areas of the world. The distribution of this disease is very tightly linked to geography with a high degree of local variation. Leishmania species, for example *L. major, L. tropica, L. guyanensis,* and *L. panamensis,* are known to infect humans and cause CL. The clinical manifestations of CL include skin lesions, which can persist for months, sometimes years. The skin lesions usually develop several weeks or months after the exposure but occasionally first appear years later. The lesions typically evolve from papules to nodular plaques to ulcerative lesions. The healing process typically results in atrophic scarring.

The *Leishmania spp.* parasite, the causal agent of leishmaniasis is transmitted by the bite of female phlebotomine sandflies. The sandflies inject the infective stage parasite, promastigotes, during blood meals. Promastigotes that reach the puncture wound are phagocytized by macrophages and transformed into amastigotes. The amastigotes multiply inside infected cells and will affect different tissues, depending in part on the *Leishmania* species, leading to the different clinical manifestations. The parasite lifecycle is completed when the sandflies become infected during blood meals ingesting amastigote infected macrophages from an infected host. In the sandfly's midgut, the parasites differentiate into promastigotes, which multiply and migrate to the proboscis.

Current treatments of VL disease rely on the use of a small group of drugs including: meglumine antimoniate, sodium stibogluconate (SSG), liposomal amphotericin B, paromomycin sulfate, and miltefosine. Although being efficacious against multiple *Leishmania* strains in different populations their use is associated with significant dose limiting adverse effects and emerging cases of drug resistance observed in specific regions. In addition, limitations related to access and need for supervised intravenous or intramuscular drug administration restricts their overall use. Miltefosine, currently the only oral drug available cannot be administered to female patients in child-bearing age due to its teratogenicity unless contraception can be guaranteed. Interestingly, there are significant differences in the clinical response to the same treatment between Asia and Africa, variations in the clinical response within Africa has also been observed leading to the development of regional treatment recommendations.

To overcome the limitations with the current drug treatments, there has been an increasing effort over recent years to discover and develop novel oral treatments with improved safety profiles. These efforts have resulted in an emerging pipeline of novel compounds in preclinical and clinical development suitable for oral treatment belonging to different chemical classes: nitroimidazoles (DNDI-0690; Antimicrob. Agents Chemother. 2019, 63, e00829-19), pyrazolopyrimidines (GSK3186899; Nature 2018, 560, 192-197), aminopyrazoles, (DNDI-1047; J. Med. Chem. 2015, 58, 9615-9624), oxaboroles (DNDI-6148; Drug Discovery Today 24, 2019, 1209-1216) and proteasome (LXE408; J. Med. Chem. 2020, 63, 10773-10781). These inhibitors represent, in cases when described, different anti-parasitic mechanisms of action which ultimately will help to mitigate the risk for resistance development if dosed as combinations.

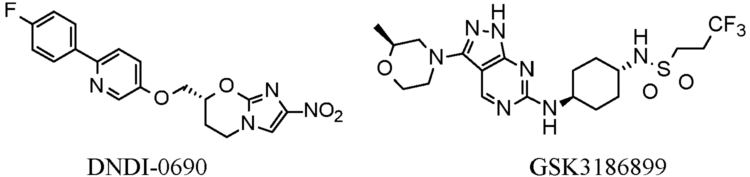

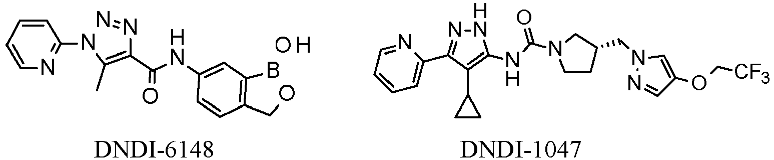

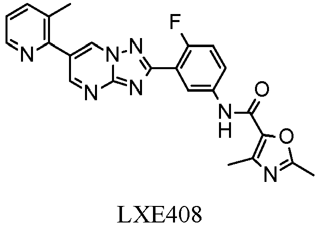

The pyrrolopyrimidine TCMDC-143610 identified by screening a set 1.8 million compounds [Peña et al. Sci. Rep. 2015, 5. https://doi.org/10.1038/srep08771.] Primarily this compound is reported to be a highly potent anti-infective activity towards *T. cruzi* infected NIH-3T3 murine fibroblasts (pIC50 6.6). TCMDC-143610 also demonstrated clearance of *L. donovani* parasites in infected human macrophages at high concentrations (pIC50 4.6).

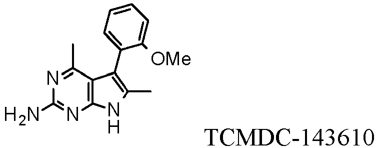

However, *in vivo* activity in *L. donovani* mouse infection models has not yet been demonstrated for this compound.

There is thus room to develop active *in vivo* compounds against parasitic diseases such as Leishmaniasis, including Visceral Leishmaniasis and Cutaneous Leishmaniasis and related diseases.

It is an aim of the present invention to provide novel compounds having an improved *in vivo* activity against parasitic diseases or neglected tropical diseases, in particular Leishmaniasis and related diseases, and to use such compounds for the treatment of these diseases.

It is a further aim to reduce, alleviate or avoid the drawbacks of the existing drugs, in particular in terms of adverse effects and drug resistance, in the treatment of parasitic diseases or neglected tropical diseases, and in particular Leishmaniasis and related diseases.

It is also an aim of the present invention to improve the access toward Leishmaniasis treatments of parasitic diseases or neglected tropical diseases, and in particular Leishmaniasis and related diseases, by providing alternative oral drugs.

It is a further object of the present invention to increase the type and number of patients susceptible to receive a treatment against parasitic diseases or neglected tropical diseases, such as Leishmaniasis and related diseases, and in particular to provide treatments adapted to female patients in child-bearing age.

### Disclosure of Invention

To this end, the present invention provides a compound of the formula (I) 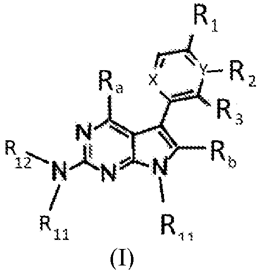 Wherein:
Rₐ and R_{b} independently denote a group selected from -CH₃ and -CF₃.
   - Both X and Y denote a carbon atom or both X and Y denote a nitrogen atom when R₂ is absent
   - R₁ denotes a group selected
      from -CN, -CF₃, halogen, -OCH₃ and -OCH₂CF₃
   - R₂ is present when Y denotes a carbon atom, and denotes:
      ∘ a hydrogen atom, or
      ∘ a group -OCH₃
   - R₃ denotes a group selected from -OCH₃, -CN, -CF₃ or halogen, or
   - R₃ forms together with the R₂ a group -OCH₂O- with the cyclic system to which they are linked,
wherein each one of R₁₁ and R₁₂ independently denotes
   ∘ a hydrogen atom

Or a pharmaceutically acceptable salt thereof, According to an embodiment, both X and Y denote a carbon atom, and the remaining groups are as defined for formula (I) or for any other embodiments here described.

According to another embodiment both X and Y denote a nitrogen atom and R₂ is absent, and the remaining groups are as defined for formula (I) or for any other embodiments here described.

According to another embodiment each one of Ra and Rb denotes a -CH₃ group and the other substituents are as defined for formula (I) of for any other embodiments here described, including formula (Ia), (Ib) and (Ic) below.

According to another embodiment, each one of Ra and Rb denotes a -CH₃ group, each one of R₁₁ and R₁₂ denotes a hydrogen atom, R₁ is selected from -CN, halogen, -OCH₃ and -OCH₂CF₃, R₂ when present, denotes a hydrogen atom or -OCH₃, R₃ is -OCH₃ or R₂ and R₃ denote together a group -OCH₂O- and the remaining substituents are as defined for formula (I) of for any other embodiments here described, including formula (Ia), (Ib) and (Ic) below.

Preferably, both Rₐ and R_{b} denote a methyl group wherein the remaining groups are as defined for formulae (I), (Ia) or for any other embodiments here described.

Preferably, the present invention provides a compound of formula (Ib) 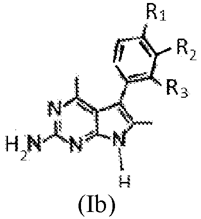 Wherein R₁, R₂ and R₃ are as above defined.

Preferably, the present invention provides a compound of formula (Ic)

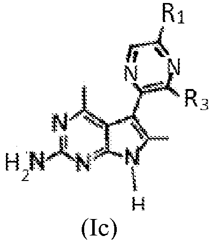

Wherein R₁ and R₃ are as above defined.

In a preferred embodiment, R₁ in formula (I), (Ia), (Ib) and (Ic) is selected among the group consisting of -CN, halogen selected from fluor or chlorine, and a group -OCH₂CF₃ and the other substituents are as above defined

In another preferred embodiment, the group R₂, in formula (I), (Ia), (Ib) and (Ic), when present, is selected among the group consisting of hydrogen and -OCH₃, or defines together with R₃ a group -OCH₂O-, and the other substituents are as above defined.

In another preferred embodiment, the group R₃ in formula (I), (Ia), (Ib) and (Ic) denotes -OCH₃ or forms together with R₂, when present a group -OCH₂O-.

The present invention specifically includes the following compounds:

| Compound | Formula |
|---|---|
| **C1** | 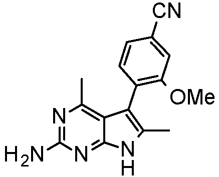 |
| 4-(2-amino-4,6-dimethyl-7H-pyrrolo[2,3-d]pyrimidin-5-yl)-3-methoxybenzonitrile | |
| **C2** | 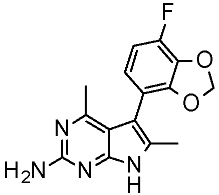 |
| 5-(7-fluoro-1,3-benzodioxol-4-yl)-4,6-dimethyl-7H-pyrrolo[2,3-d]pyrimidin-2-amine | |
| **C3** | 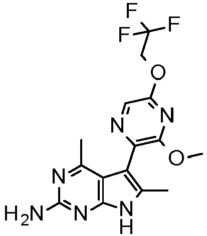 |
| 5-[3-methoxy-5-(2,2,2-trifluoroethoxy)pyrazin-2-yl]-4,6-dimethyl-7H-pyrrolo[2,3-d]pyrimidin-2-amine | |
| **C4** | 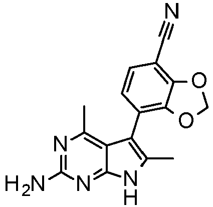 |
| 7-(2-amino-4,6-dimethyl-7H-pyrrolo[2,3-d]pyrimidin-5-yl)-1,3-benzodioxole-4-carbonitrile | |

In the present disclosure, the term "alkyl" denotes a saturated hydrocarbon chain having the indicated number of carbon atoms.

The term "alkoxy" denotes a group -O-alkyl.

The term "alkenyl" denotes a hydrocarbon chain having the indicated number of carbon atoms and comprising at least one double bound, up to the maximal possible double bounds with regards to the indicated number of carbon atoms.

The term "alkynyl" denotes a hydrocarbon chain having the indicated number of carbon atoms and comprising at least one triple bound, up to the maximal possible triple bounds with regards to the indicated number of carbon atoms. An alkenyl may in addition comprise one or several double bounds.

The term "cyclic" refers to a chain arrangement of atoms forming at least one ring, with some, or all, of the atoms constituting the chain arrangement. A cyclic ring may comprise one or several unsaturated bonds such as double or triple bounds. Cyclic alkenyl may include several double bounds and form aromatic rings.

The term "substituent" refers to an atom or atom arrangement replacing one or several hydrogen atoms on the indicated hydrocarbon chain.

The compounds of formula (I) and embodiments here-described may exist in enantiomeric forms. It is to be understood that all enantiomers, diastereomers, racemates and mixtures thereof are included within the scope of the present invention.

Compounds of formula (I) and embodiments here-described may in addition or alternatively exist in various tautomeric forms. All tautomeric forms and mixtures thereof are included within the scope of the present invention.

The present invention includes compounds of formula (I) and embodiments here-described in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulfonic and benzenesulfonic acids.

According to a particular aspect, the present disclosure provides the use of the compounds of Formulae (I), (Ia) or related embodiments here-described or a pharmaceutically acceptable salt thereof, as a medicament. The present disclosure thus relates to a compound of Formulae (I), (Ia) or related embodiments here-described or a pharmaceutically acceptable salt thereof for use as a medicament.

According to another aspect, the present disclosure provides the use of the compounds of Formulae (I), (Ia) or related embodiments here-described or a pharmaceutically acceptable salt thereof, in the treatment or prophylaxis of parasitic diseases. The present disclosure thus relates to a compound of Formulae (I), (Ia) or related embodiments here-described or a pharmaceutically acceptable salt thereof for use in the treatment of parasitic diseases. The compound of the present disclosure is thus understood as being used for the manufacture of a medicament.

The pharmaceutically acceptable salts here mentioned are those commonly used in the pharmaceutical field. They include for example the salts derived from organic or inorganic pharmaceutical acids such as adipate, bisulfate, camphorate, acetate, citrate, benzoate, sulfonate, succinate, propionate, phosphate as well as related derivatives and mixture thereof.

The parasitic diseases preferably denotes tropical parasitic diseases and more preferably neglected tropical diseases. The parasitic diseases thus include malaria, caused for example by one of *plasmodium falciparum, plasmodium vivax, plasmodium ovale, plasmodium malariae* and Leishmaniasis, caused for example by *L. major, L. tropica, L. guyanensis, L. panamensis, L. donovani, L. chagasi, L. infantum, L. mexicana, L. amazonesis, L. venezuelensis, L. braziliensis, L. peruviana, L. aetiopica,* parasites, or of subgenius of *Viannia,*
In a most preferred embodiment, the present disclosure relates to a compound of Formulae (I), (Ia) or related embodiments here-described or a pharmaceutically acceptable salt thereof for use in the treatment or prophylaxis of *Leishmania spp.* dependent disease conditions. This includes Visceral Leishmaniasis, Cutaneous Leishmaniasis, and any *Leishmania* dependent symptoms and complications such as splenomegaly, irregular fevers, anaemia, pancytopenia, weight loss, skin lesion, internal organ lesions, papules or nodular plaques, ulcerative lesions and weakness.

According to another aspect, the present compounds Formulae (I), (Ia) or related embodiments here-described or a pharmaceutically acceptable salt thereof are used alone or in combination with one or more known drugs, such as meglumine antimoniate, sodium stibogluconate (SSG), liposomal amphotericin B, paromomycin sulfate, and miltefosine.

The present compounds Formulae (I), (Ia) or related embodiments here-described or a pharmaceutically acceptable salt thereof may be used or administered in combination with one or more other treatment selected from the group consisting of hydroxychloroquine, chloroquine, quinine sulfate, doxycycline, tetracycline, clindamycin, primaquine, and quinidine gluconate.

When administered in combination with another known drug, or another combination of known drugs, the compounds of the present disclosure may be physically combined with such known drugs into an appropriate form such as a pill, a topical formulation, an injectable solution or any other suitable form. Alternatively, the compound of the present disclosure may be used in combination with another known drugs in a form different from the one of the combined drug or drugs.

When combined to another medical treatment, the compounds here-disclosed may be administer to the patient either simultaneously, alternatively, or consequently to such other treatments.

Alternatively or in addition, the compounds of the present disclosure may be used in combination with drugs used for other medical indications. For example, the compounds of the present invention may be used in combination with drugs used as antiinflammatory, anti-coagulant, antibiotics, anti-cancers, or for immune related diseases.

For the above-mentioned therapeutic indications, the dose of the compound to be administered may depend on different parameter such as the specific compound employed, the specific disease being treated, in particular whether it related to Visceral Leishmaniasis or Cutaneous Leishmaniasis, the mode of administration, the age, weight and sex of the patient. Such factors may be determined by the attending physician.

The administration may be done once or twice a day, or up to 6 times a day, on a period of time comprised between few days such as 3 or 4 days or a week up to several months such as two, three or four months. The dosage regimen may depend on whether it related to preventive or curative application.

**In** general, satisfactory results are obtained when the compounds are administered to a human at a daily dosage of between 0.1 mg/kg to 100 mg/kg (measured as the active ingredient).

According to another aspect, the present disclosure relates to a pharmaceutical composition comprising the compounds here-described. **In** particular, compounds of formula (I) and related embodiments may be used on their own, as a combination with another pharmacologically active ingredient, or in the form of appropriate pharmaceutical formulations comprising the compound of the invention or a salt thereof in combination with a pharmaceutically acceptable diluent, buffer, adjuvant, excipient or carrier.

Particularly preferred are compositions not containing material capable of causing an adverse reaction, for example, an allergic reaction. The pharmaceutical composition may be adapted for an acceptable mode of administration such as rectal, buccal, topical, intranasal and transdermal administration. The formulation thus relates to one of the routes including intra-arterial injection, intravenous injection, intraperitoneal injection, parenteral injection, intramuscular injection, subcutaneous injection, oral, topical or inhalant. Depending on the selected route of administration, the present pharmaceutical formulation may take the form of an aqueous suspension, oil suspension, emulsion, tablet, pill, powder, elixir, solution, syrup, aerosol or capsule. The formulation may be adapted for quick release of the compound of the present invention or for delayed released or for sustained release. It may thus comprise adapted carriers such as nanoparticles, zeolites, polymer matrix assemblies, polymer coating and related additives.

According to another aspect, the present invention also provides a kit comprising the compounds of formula (I) or of related embodiments here-described. Such a kit further includes instructions regarding the use or administration of the compound. Instructions may be printed or accessible through a website. Instruction comprises at least the name of the compound and the dosage. It may in addition comprise conditions for prophylaxis or treatment of one parasitic disease mentioned above. The kit may in addition comprise a device adapted for the administration of the compound of the present disclosure according to the instructions, such as an injection device, or an auto-injection device, either manual or automatized.

According to an aspect, the patient is a human patient, either male or female. According to another aspect, the disclosed compound is administered to non-human mammals including an animal model, a domestic animal such as cows, sheep, cats, dogs, pigs, rabbits, or a wild animal such as rats, mice, or monkeys. The human patient or the non-human mammal may be healthy and considered at risk of being infected by one of the above-mentioned diseases. Alternatively, the human patient or the non-human mammal may be already infected by one of the above-mentioned diseases and in need of an appropriate treatment. Alternatively or in addition, the human patient or the non-human mammal may experience drug resistance in the treatment of one of the above-mentioned disease and in need of an alternative or combined treatment.

In a further aspect the invention provides a process for the preparation of a compound of formula (l), (Ia) or related embodiments here-described. The process comprises the step of reacting an Intermediate of formula (II): 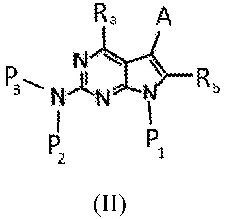
wherein Rₐ and R_{b} are as above described for formula (I), (Ia) or the related embodiments and wherein :
   - A denotes a group selected from I, Br, or a group dioxaborolan-2-yl;
   - P₁, P₂ and P₃ independently denote an hydrogen atom or a protective group selected from a terbutoxycarbonyl (Boc), 2-(trimethylsilyl)ethoxymethyl (SEM), a paramethoxybenzyl (PMB); or one of the groups R₁₁ and R₁₂;
with an intermediate of Formula (III) 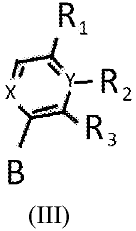
wherein X, Y, R₁, R₂ and R₃ are as above-defined for formulae (I), (Ia) and related embodiments and wherein B denotes:
   - a group dioxaborolan-2-yl when A denotes I or Br,
   - or I or Br when A denotes dioxaborolan-2-yl;
in the presence of a Palladium based catalyst such as Pd(PPh₃)₄ or Pd(dppf)Cl₂, and a carbonate such as Na₂CO₃ or K₂CO₃, or a phosphate such as K₃PO₄, at a temperature comprised between 60°C and 150°C, preferable around 100°C to 120°C in an aqueous solvent comprising dioxane or DMSO or a mixture thereof.

The coupling reaction between the intermediate of Formula (II) and the intermediate of Formula (III) results in a pyrrolo[2,3-d]pyrimidin-2-amine derivative of Formula (IV).

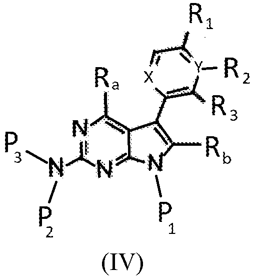

Wherein P₁, P₂ and P₃ are as defined for the intermediate of Formula (II) and wherein X, Y, R₁, R₂ and R₃ are as above-defined for formulae (I), (Ia) and related embodiments.

The process may optionally comprise a further step of reacting the resulting pyrrolo[2,3-d]pyrimidin-2-amine derivative of Formula (IV) with suitable derivatives under suitable reaction condition so as to replace, where necessary, the groups P₁, P₂ and P₃ with another group corresponding to R₁₁ and R₁₂ in such a way to obtain a compound of formula (I), (Ia) or related embodiment. This further step may comprise several successive appropriate reaction steps. In particular, the process comprises such a further step when at least one of P₁, P₂ and P₃ is different from the expected group R₁₁ and R₁₂. The suitable derivatives and reaction conditions may be determined by the skilled practitioner according to the nature of the groups R₁₁ and R₁₂.

### General methods

All reagents and solvents were used as received from the manufacturer. All reactions were performed open to air unless an inert atmosphere is noted.

Analytical thin layer chromatography (TLC) was performed using Sil G/UV 254 aluminium plates and visualized with the aid of UV light if not otherwise noted.

Normal phase flash column chromatography was conducted on either an Argonaut Flashmaster II system or a Grace Reveleris X2 Flash Instrument using disposable cartridges packed with silica gel 60 (0.040-0.063 mm, 230-400 mesh ASTM). Reverse phase chromatography was conducted on a Grace Reveleris X2 Flash Instrument using disposable C18 cartridges with variable water/acetonitrile gradients (acidic modifier 0.1% trifluoroacetic acid was typically used). Chromatography grade solvents were used in both cases.

Analytical high performance liquid chromatography was performed on an Agilent 1100 HPLC system fitted with a Quaternary Pump, a Diode Array Detector (UV), and an Agilent Zorbax XDB-C8 column (4.6 × 75 mm, 3.5µm). A typical gradient of 5% to 100% acetonitrile (+ 0.1% TFA) in water (+ 0.1% TFA) was used.

LC/MS analysis was performed on an Agilent 6120 Quadrupole LCMS system (ESI scanning mode, both positive and negative ions of 150 to 700 amu detected with the standard method) with sample delivery via an Agilent 1260 Infinity HPLC with Quaternary Pump and Variable Wavelength Detector (UV). Column: Agilent ZORBAX SB-C8, 4.6 x 30 mm, 3.5 micron. A gradient of 10% to 100% of acetonitrile (+ 0.1% formic acid) in water (+ 0.1% formic acid) at flow rate of 1 mL/min was used for the HPLC separation.

NMR spectra were recorded on a Bruker 400 Avance III Nanobay spectrometer. NMR spectra are reported in ppm with reference to an internal tetramethylsilane (TMS) standard (0.00 ppm for 1H and 13C) or residual solvent resonances as reported in doi.org/10.1021/om100106e. When peak multiplicities are reported, the following abbreviations are used: s = singlet, d = doublet, t = triplet, q = quartet, m = multiplet, br = broadened, dd = doublet of doublets, dt = doublet of triplets, bs = broadened singlet. Coupling constants, when given, are reported in hertz (Hz).

### Preparation of Synthetic Intermediates

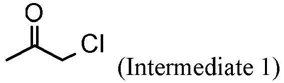

### Intermediate 1: Chloroacetone

To a 1 L round bottom flask was added ethyl-2-chloroacetoacetate (150 g, 911.4 mmol) and water (300 ml). The round bottom flask was then placed in a water bath (heat sink) after which sulfuric acid (150 ml) was added slowly over ~15 min. The reaction was then heated to 110°C for 1.5 h until emission of CO2 ceased, the reaction mixture was cooled, and the product extracted into DCM (100 ml, 3x50 ml). The organic layers were combined, washed with water (2x100 ml), sat. aqueous NaCl (100 ml), dried over magnesium sulphate, filtered and evaporated on a rotary evaporator (room temperature water bath) to give chloroacetone **(Intermediate** 1) (60.26 g, 71%) as a light-brown liquid.

¹H-NMR (400 MHz, CDCl₃): 2.31 (s, 3H, CH₃), 4.10 (s, 2H, CH₂).

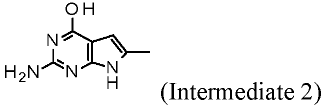

### Intermediate 2: 2-amino-6-methyl-7H-pyrrolo[2,3-d]pyrimidin-4-ol

To a 2 L round bottom flask was added 2,4-diamino-6-hydroxypyrimidine (45.0 g, 356.8 mmol), sodium acetate (82.0 g, 356.8 mmol) and water (800 ml). The suspension was then heated to 100°C for 10 min after which **Intermediate** 1 was added slowly over 30 min from a dropping funnel. After a further 3 h at 100°C, the reaction mixture was cooled to room temperature. The resulting precipitate was collected by filtration, washed with water (2x100 ml), dried under reduced pressure (60°C on a rotary evaporator), then under high vacuum with phosphorus pentoxide to constant weight to give 2-amino-6-methyl-7H-pyrrolo[2,3-d]pyrimidin-4-ol **(Intermediate** 2) (47.3 g, 81%) as a beige solid.

LCMS: Rt 1.28 min, m/z: [M+H]+ Calcd for C₇H₈N₄O 165.08; Found 165.10.

¹H-NMR (400 MHz, DMSO-d6): 2.15 (d, 3H, CH₃, J 0.8Hz), 2.86 (q, 1H, ArH, J 0.8Hz), 5.99 (bs, 2H, NH₂), 10.17 (bs, 1H, NH/OH), 10.80 (bs, 1H, NH/OH).

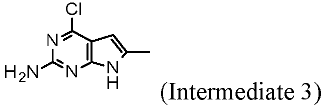

### Intermediate 3: 4-chloro-6-methyl-7H-pyrrolo[2,3-d]pyrimidin-2-amine

To a 1 L round bottom flask were added **Intermediate** 2 (25.0 g, 152.3 mmol), phosphorus oxychloride (250 ml) and N,N-dimethylaniline (0.69 ml, 5.5 mmol). The mixture was heated to reflux for 3 h after which time the reaction mixture was cooled and excess phosphorus oxychloride was removed under reduced pressure. The remaining material was carefully poured onto 500 ml crushed ice then basified with 25% aqueous ammonia (~400 ml). The resulting precipitate was collected by filtration and dried under high vacuum with phosphorus pentoxide to constant weight to give 4-chloro-6-methyl-7H-pyrrolo[2,3-d]pyrimidin-2-amine **(Intermediate** 3) (24.55 g, 88%) as a light brown solid.

LCMS: Rt 2.45 min, m/z: [M+H]+ Calcd for C₇H₇ClN₄ 183.04; Found 183.10.

¹H-NMR (400 MHz, DMSO-d6): 2.27 (d, 3H, CH₃, J 0.8Hz), 5.93 (q, 1H, ArH, J 0.8Hz), 6.35 (bs, 2H, NH₂), 11.42 (bs (1H, NH).

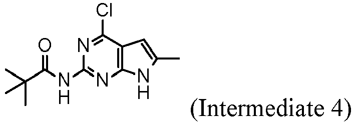

### Intermediate 4: N-(4-chloro-6-methyl-7H-pyrrolo[2,3-d]pyrimidin-2-yl)-2,2-dimethylpropanamide

To a solution of **Intermediate** 3 (90.0 g, 492.9 mol) in pyridine in a 2 L round bottom flask, under a nitrogen atmosphere, was added trimethylacetyl chloride (72.8 ml, 591.4 mol) slowly from a nitrogen-purged dropping funnel over a period of 1 h. After 2 h, the pyridine was thoroughly evaporated to give a thick resin to which ~300 ml water was added. The product was manipulated to a solid, collected by filtration with suction and washed with water (3x100 ml). After air drying overnight, the product was transferred to a 500 ml beaker and dried under high vacuum with phosphorus pentoxide until a constant weight was obtained (~3 days), to give N-(4-chloro-6-methyl-7H-pyrrolo[2,3-d]pyrimidin-2-yl)-2,2-dimethylpropanamide **(Intermediate** 4) (121.8 g, 93%) as a brown solid.

LCMS: Rt 3.67 min, m/z: [M+H]+ Calcd for C₁₂H₁₅ClN₄O 267.10; Found 267.10.

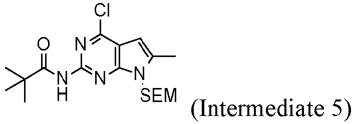

### Intermediate 5: N-(4-chloro-7-SEM-6-methyl-7H-pyrrolo[2,3-d]pyrimidin-2-yl)-2,2-dimethylpropanamide

To a 2 L, nitrogen-purged round bottom flask was added **Intermediate 4** (100 g, 374.9 mmol) and anhydrous dimethylformamide (500 ml). After cooling in an ice bath, sodium hydride (60% in mineral oil) (18.00 g, 449.9 mol) was added portion wise over ~10 min then stirred for a further 10 min before the dropwise addition of 2-(trimethylsilyl)ethoxymethyl chloride (79.8 ml, 449.9 mol) via a nitrogen-purged dropping funnel over ~20 min. After stirring on ice for 1 h, the reaction mixture was quenched with water (500 ml) (add slowly) then transferred to a separating funnel (2 L). The product was extracted with EtOAc (300 ml, 4x100 ml). The organic layers were combined, back-washed with water (2x100 ml), dried over MgSO₄, filtered and evaporated. The resulting oil was dissolved in hexane (300 ml) then poured onto a 18.5 cm wide, 5 cm long hexane-wet silica plug and eluted with 0:100 to 30:70 EtOAc:hexane (over ~3-4 L). Fractions containing product (by TLC) were evaporated in a 2 L round bottom flask to give N-(4-chloro-7-SEM-6-methyl-7H-pyrrolo[2,3-d]pyrimidin-2-yl)-2,2-dimethylpropanamide **(Intermediate 5)** (122.7 g, 82%) as a red oil.

LCMS: Rt 4.94 min, m/z: [M+H]+ Calcd for C₁₈H₂₀ClN₄O₂S1 397.18; Found 397.20.

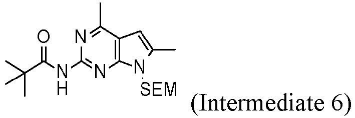

### Intermediate 6: N-(7-SEM-4,6-dimethyl-7H-pyrrolo[2,3-d]pyrimidin-2-yl)-2,2-dimethylpropanamide

The 2 L round bottom flask containing **Intermediate 5** (122.7 g, 309.1 mmol) was sealed then purged with nitrogen before addition of anhydrous tetrahydrofuran (600 ml), 1-methyl-2-pyrrolidinone (89.2 ml, 927.2 mol) and iron(III) acetylacetonate (10.91 g, 30.9 mmol). The seal was replaced with a nitrogen-purged dropping funnel from which methyl magnesium bromide (3 M in ether) (206.1 ml, 618.2 mmol) was added dropwise over ~1 h. After a further 15 min, 2M HCl (300 ml) was added (caution: add the first ~50 ml very slowly). The product was then extracted with EtOAc (200 ml, 3x100 ml) (add extra 2M HCl to the separating funnel if a gel appears). The organic layers were combined, back-washed with water (2x150 ml), dried over MgSO₄, filtered and evaporated. The resulting oil was dissolved in DCM (300 ml) then poured onto a 18.5 cm wide, 5 cm long hexane-wet silica plug and eluted with 0:100 to 0:100 EtOAc:hexane (over ~3 L) then 0:100 to 10:90 MeOH:EtOAc (~200 ml fractions). Fractions containing product (by TLC) were evaporated in a 2 L round bottom flask to give N-(7-SEM-4,6-dimethyl-7H-pyrrolo[2,3-d]pyrimidin-2-yl)-2,2-dimethylpropanamide **(Intermediate 6)** (105.3 g, 90%) as a red oil.

LCMS: Rt 3.92 min, m/z: [M+H]+ Calcd for C₁₉H₃₂N₄O₂Si 377.24; Found 377.25.

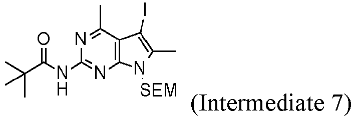

### Intermediate 7: N-(5-iodo-4,6-dimethyl-7-SEM-7H-pyrrolo[2,3-d]pyrimidin-2-yl)-2,2-dimethylpropanamide

To a solution of **Intermediate 6** (105.8 g, 279.6 mmol) in dimethylformamide (non-anhydrous) (400 ml) was added N-iodosuccinimide (66.06 g, 293.6 mmol) portionwise over 10 min. After 1 h, the reaction was diluted with water (1 L) and treated with sodium meta-bisulfite (portion-wise until decolored from iodine). The product was extracted with EtOAc (300 ml, 4x100 ml). The organic layers were combined, back-washed with 5% sodium bicarbonate (150 ml), water (2x150 ml), sat. aqueous NaCl (150 ml), dried over MgSO₄, filtered then evaporated. The resulting solid was dissolved in DCM (200 ml) and poured onto a 18.5 cm wide, 5 cm long hexane-wet silica plug then eluted with 0:100 to 50:50 EtOAc:hexane (over ~5-7 L). Fractions containing product (by TLC) were evaporated in a 2 L round bottom flask to give N-(5-iodo-4,6-dimethyl-7-SEM-7H-pyrrolo[2,3-d]pyrimidin-2-yl)-2,2-dimethylpropanamide **(Intermediate** 7) (112.4 g, 80%) as a cream solid.

LCMS: Rt 4.75min, m/z: [M+H]+ Calcd for C₁₉H₃₁IN₄O₂Si 503.13; Found 503.10.

¹H-NMR (400 MHz, CDCl₃): -0.08 (s, 9H, 3xCH₃), 0.93-0.98 (m, 2H, CH₂), 1.34 (s, 9H, 3xCH₃), 2.51 (s, 3H, CH₃), 2.90 (s, 3H, CH₃), 3.50-3.54 (m, 2H, CH₂), 5.64 (s, 2H, CH₂), 8.01 (s, 1H, NH).

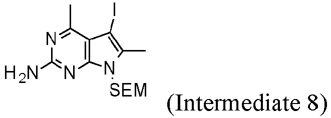

### Intermediate 8: 7-SEM-5-iodo-4,6-dimethyl-7H-pyrrolo[2,3-d]pyrimidin-2-amine

To a 1 L round bottom flask was added **Intermediate** 7 (30.0 g, 59.71 mmol), MeOH (200 ml) and 2 M sodium hydroxide (60 ml). After heating at reflux for 3 h, the reaction was concentrated to about half the volume then diluted with water (200 ml). After cooling in an ice bath, the resulting precipitate was collected by filtration and dried under high vacuum with phosphorus pentoxide to constant weight to give 7-SEM-5-iodo-4,6-dimethyl-7H-pyrrolo[2,3-d]pyrimidin-2-amine **(Intermediate 8)** (24.24 g, 97%) as a straw-yellow solid.

LCMS: Rt 3.94 min, m/z: [M+H]+ Calcd for C₁₄H₂₃IN₄OSi 419.08; Found 419.10.

¹H-NMR (400 MHz, CDCl₃): -0.05 (s, 9H, 3xCH₃), 0.88-0.92 (m, 2H, CH₂), 2.44 (s, 3H, CH₃), 2.79 (s, 3H, CH₃), 3.48-3.53 (m, 2H, CH₂), 4.81 (bs, 2H, NH₂), 5.47 (s, 2H, CH₂).

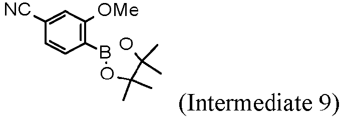

### Intermediated 9: 5-cyano-2-methoxyphenylboronic acid pinacol ester

To a 250 ml, oven dried round bottom flask was added 4-bromo-3-methoxybenzonitrile (15.0 g, 70.74 mmol), bis(pinacolato)diboran (26.95 g, 106.11 mmol) and potassium acetate (20.83 g, 212.22 mmol). After purging with nitrogen, anhydrous dioxane was added and the solution was bubbled with nitrogen for ~10 min, while stirring, before addition of Pd(dppf)Cl₂ (2.59 g, 3.43 mmol). After heating at 100°C for 2 h, the reaction was quenched with water (200 ml) and extracted with EtOAc (200 ml, 4x50 ml). The organic layers were combined, dried over MgSO₄, filtered then concentrated to ~50 ml on a rotary evaporator. The oil was diluted with hexane (50 ml) and poured onto a 9 cm wide, 6 cm long, hexane-wet silica plug and eluted with 0:100 to 20:80 EtOAc:hexane (~200 ml fractions). Fractions containing product (by TLC) were evaporated to give 5-cyano-2-methoxyphenylboronic acid pinacol ester **(Intermediated** 9) (17.63 g, 96%) as a white solid.

LCMS: Rt 3.08 (boronic acid), 4.47 (pinacol ester) min, m/z: [M-H]- Calcd for C₈H₈BNO₃ 176.05 (boronic acid fragment); Found 176.10.

¹H-NMR (400 MHz, CDCl₃): 1.36 (s, 12H, 4xCH₃), 3.86 (s, 3H, CH₃), 7.06 (d, 1H, ArH, J 1.2Hz), 7.23 (dd, 1H, ArH, J 7.6Hz, 1.2Hz), 7.72 (d, 1H, ArH, J 7.6Hz).

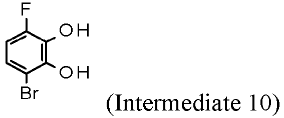

### Intermediate 10: 3-bromo-6-fluorobenzene-1,2-diol

To a 500 ml oven-dried round bottom flask was added 2-bromo-5-fluorophenol (15.0 g, 78.53 mmol), anhydrous MgCl₂ (14.95 g, 157.07 mmol) and paraformaldehyde (9.43 g, 314.14 mmol). The round bottom flask was then sealed and purged with nitrogen before addition of anhydrous tetrahydrofuran (150 ml). The seal was replaced with a nitrogen-purged dropping funnel from which triethylamine (21.77 ml, 157.07 mmol) was added slowly over five minutes. The reaction was then heated to 75°C for 10 h after which the reaction was immersed in a water bath (heat sink) before the slow addition of sodium hydroxide (6.28 g, 157.07 mmol) in water (100 ml) followed by the dropwise addition of hydrogen peroxide (30% in water) (32 ml), via a dropping funnel, over 15 min. After 2 h, the reaction was acidified with 32% HCl to pH ~1 then extracted with ether (200 ml, 4 x 50 ml). The combined organic layers were washed with 5% sodium sulfite (2 x 100 ml), saturated NaCl (100 ml), dried over MgSO₄, filtered, evaporated on silica (~15 g) then subjected to flash chromatography with 0:100 to 50:50 EtOAc:hexane (80 g silica). Fractions containing desired product (TLC) were evaporated to give 3-bromo-6-fluorobenzene-1,2-diol **(Intermediate 10)** (5.93 g, 37%) as a yellow oil.

LCMS: Rt 2.40 min, m/z: [M-H]- Calcd for C6H4BrFO2 204.93; Found 205.00.

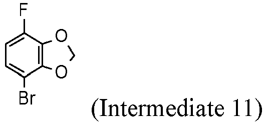

To a 500 ml round bottom flask was added **Intermediate 10** (9.60 g, 46.38 mmol), cesium carbonate (22.67 g, 69.57 mmol) and dimethylformamide (70 ml). The reaction was allowed to stir for 5 minutes while purging with nitrogen before addition of chloroiodomethane (2.51 ml, 34.38 mmol). The reaction was then heated to 80°C for 1 h after which the dimethylformamide was evaporated under reduced pressure. The resulting residue was partitioned between EtOAc (200 ml) and water (100 ml). The organic layer was separated and washed with 5% sodium carbonate (50 ml), water (3 x 50 ml), saturated NaCl (50 ml), dried over MgSO₄, filtered, evaporated on silica (~10 g) then subjected to flash chromatography with 0:100 to 30:70 EtOAc:hexane (80g silica). Fractions containing product were evaporated to give 4-bromo-7-fluoro-1,3-benzodioxole **(Intermediate 11)** (4.34 g, 43%) as a white solid.

GCMS: Rt 9.77 min, m/z: [M]+ Calcd for C13H16BFO4 217.94; Found 218.

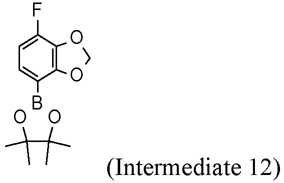

### Intermediate 12: 7-fluoro-1,3-benzodioxole-4-boronic acid pinacol ester

To a 250 ml, oven dried round bottom flask was added **Intermediate 11** (6.39 g, 29.18 mmol), bis(pinacolato)diboran (8.15 g, 32.09 mmol) and potassium acetate (8.59 g, 87.53 mmol). After purging with nitrogen, anhydrous dioxane was added then bubbled with nitrogen for ~10 min, while stirring at room temp, before addition of Pd(dppf)Cl₂ (2.59 g, 3.43 mmol). After heating at 100°C for 8 h, the reaction was quenched with water (200 ml) and extracted with EtOAc (100 ml, 4 x 30 ml). The organic layers were then combined, washed with saturated NaCl (50 ml), dried over MgSO₄, filtered, evaporated on silica (~15 g) then subjected to flash chromatography with 0:100 to 50:50 EtOAc:hexane (80 g silica). Fractions containing product (TLC) were evaporated to give 7-fluoro-1,3-benzodioxole-4-boronic acid pinacol ester **(Intermediate 12)** (5.94 g, 77%) as a white solid.

GCMS: Rt 13.32 min, m/z: [M]+ Calcd for C13H16BFO4 266.11; Found 266.

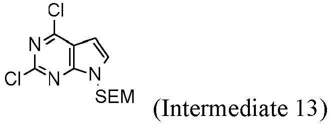

To a solution of compound 2,4-dichloro-7H-pyrrolo[2,3-d]pyrimidine (1.40 kg, 7.45 mol, *1 eq)* in THF (21 L) was cooled to 0-10°C. The mixture was added NaH (387 g, 9.68 mol, 60% purity, 1.3 *eq)* in portions, then, the mixture was stirred for 0.5 h at 0-10°C. SEM-Cl (1.61 kg, 9.68 mol, 1.71 L, 1.3 *eq)* was added drop wise into the mixture and stirred for 1 h. TLC (petroleum ether:EtOAc 5:1, product Rf 0.8) showed the starting material was consumed completely. The mixture was poured into NH₄Cl(aq) (10 L) solution and extracted with MTBE (5 L × 2). The combined organic phase was washed with brine (2 L × 3), dried over with Na₂SO₄, filtered and concentrated in vacuum. The residue was purified by column chromatography (silica, petroleum ether:EtOAc 1:0 to 60:1) to obtain compound 2,4-dichloro-7-SEM-pyrrolo[2,3-d]pyrimidine **(Intermediate** 13) (2.70 kg, crude) as light-yellow oil.

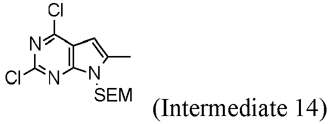

### Intermediate 14: 2,4-dichloro-6-methyl-7-SEM-pyrrolo[2,3-d]pyrimidine

To a solution of compound **Intermediate 13** (1.60 kg, 5.03 mol, 1 eq) in THF (20 L) was cooled to -78°C, and then, LDA (2 M, 3.02 L, 1.2 eq) was added drop wise into the mixture. After stirred for 0.5 h, CH₃I (2.14 kg, 15.0 mol, 938 mL, 3 eq) was added into the mixture, and stirred for 1 h at -78°C. LCMS (product: Rt 0.993 min) showed the starting material was consumed completely and desired m/z was detected. The reaction mixture was quenched with NH₄Cl(aq) solution (10 L), diluted with EtOAc (3 L). The aqueous phase was extracted with EtOAc (2 L × 2). The combined organic phase was washed with brine (2 L × 2), dried with Na₂SO₄, filtered and concentrated in vacuum. 2,4-dichloro-6-methyl-7-SEM-pyrrolo[2,3-d]pyrimidine **(Intermediate 14)** (1.70 kg, crude) was obtained as red oil.

LCMS: Rt 0.993 min, m/z 332 [M+1]+.

¹H-NMR (400 MHz, CDCl₃): 6.36 (s, 1 H), 5.59 (s, 2 H), 3.56 - 3.50 (m, 2 H), 2.53 (s, 3 H), 0.86 - 0.88 (m, 2 H), 0.04 (s, 9 H).

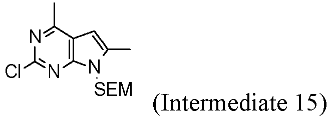

### Intermediate 15: 2-Chloro-4,6-dimethyl-7-SEM-pyrrolo[2,3-d]pyrimidine

To a solution of compound **Intermediate 14** (1.70 kg, 5.12 mol, 1 eq) in THF (20 L) was added Fe(ACAC)₃ (722 g, 2.05 mol, 0.4 eq). The mixture was cooled to -78°C, and MeMgBr (3 M, 4.26 L, 2.5 eq) was added drop wise into the mixture. The mixture was stirred for 2 h at -78°C. TLC (petroleum ether:EtOAc 10:1, product Rf 0.5) showed the starting material was consumed completely. The reaction mixture was quenched with NH₄Cl(aq) (8 L), diluted with EtOAc (4 L). The aqueous phase was extracted with EtOAc (2 L × 5). The combined organic phase was washed with brine (2 L × 3), dried with Na₂SO₄, filtered and concentrated in vacuum. The residue was purified by column chromatography (silica, petroleum ether:EtOAc 50:1 to 10:1) to obtain compound 2-chloro-4,6-dimethyl-7-SEM-pyrrolo[2,3-d]pyrimidine **(Intermediate 15)** (1.18 kg, 3.78 mol, 73.9% yield) as yellow oil.

LCMS: Rt 0.935 min, m/z 312 [M+1]+.

¹H-NMR (400 MHz, CDCl₃): 6.30 (s, 1 H), 5.59 (s, 2 H), 3.53 - 3.49 (m, 2 H), 2.67 (s, 3 H), 2.51 (s, 3 H), 0.96 - 0.87 (m, 2 H), 0.05 (s, 9 H)

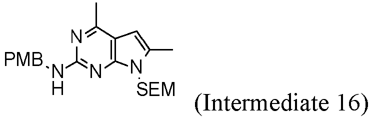

### Intermediate 16: N-[(4-methoxyphenyl)methyl]-4,6-dimethyl-7-SEM-pyrrolo[2,3-d]pyrimidin-2-amine

A mixture of compound **Intermediate 15** (666 g, 2.14 mol, 1 eq) and PMBNH₂ (2.93 kg, 21.3 mol, 2.76 L, 10 eq) was stirred at 140°C for 12 h. TLC (petroleum ether:EtOAc 5:1, product Rf 0.4) showed the starting material was consumed completely, and desired m/z was detected by LCMS (product: Rt 0.822 min). The reaction mixture was washed with 1 M HCl solution (2 L × 4) and extracted with EtOAc (2 L × 3). The combined organic phase was adjusted pH to 7~8 with NaHCO₃(aq) solution and the washed with brine (2 L × 3), dried with Na₂SO₄, filtered and concentrated in vacuum. The crude product was triturated with petroleum ether at 25°C for 30 min, then, the mixture was filtered and the filter cake was concentrated in vacuum to obtain N-[(4-methoxyphenyl)methyl]-4,6-dimethyl-7-SEM-pyrrolo[2,3-d]pyrimidin-2-amine **(Intermediate 16**)(800 g, 1.94 mol, 90.80% yield) as yellow solid.

LCMS: Rt 0.822 min, m/z 413 [M+1]+.

¹H-NMR (400 MHz, CDCl₃): 7.32 - 7.29 (m, 2 H), 6.86 - 6.83 (m, 2 H), 6.09 (d, J 1.2 Hz, 1 H), 5.47 (s, 2 H), 5.15 (t, J 6.0 Hz, 1 H), 4.60 (d, J 5.6 Hz, 2 H), 3.80 (s, 3 H), 3.50 (dd, J 8.4, 7.6 Hz, 2 H), 2.51 (s, 3 H), 2.41 (s, 3 H), 0.91 - 0.85 (m, 2 H), 0.05 (s, 9 H).

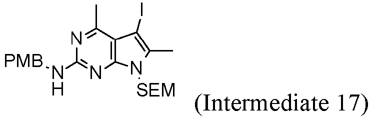

### 5-iodo-N-[(4-methoxyphenyl)methyl]-4,6-dimethyl-7-SEM-pyrrolo[2,3-d]pyrimidin-2-amine

To a solution of compound **Intermediate 16** (182 g, 441 mmol, 1 eq) in DMF (1.8 L) was added NIS (99.2 g, 441 mmol, 1.0 eq) portion wise. The mixture was stirred for 1 h at 0°C. TLC (petroleum ether:EtOAc 3:1, product Rf 0.6) showed the starting material was consumed completely. The mixture was poured into water (4 L) and extracted with EtOAc (1 L × 3). The combined organic phase was washed with brine (1 L × 3), dried with Na₂SO₄, filtered and concentrated in vacuum. The residue was purified by column chromatography (silica, petroleum ether:EtOAc 15:1 to 10:1) to obtain compound 5-iodo-N-[(4-methoxyphenyl)methyl]-4,6-dimethyl-7-SEM-pyrrolo[2,3-d]pyrimidin-2-amine **(Intermediate 17**) (840 g, 1.51 mol, 96.6% purity) as yellow solid.

LCMS: Rt 0.969 min, m/z 539 [M+1]+.

HPLC: Rt 3.236 min, purity 96.6%.

¹H-NMR (400 MHz, CDCl₃): 7.27 (d, J 8.4 Hz, 2 H), 6.83 - 6.80 (d, J 8.4 Hz, 2 H), 5.46 (s, 2 H), 5.18 (t, J 5.6 Hz, 1 H), 4.55 (d, J 6.0 Hz, 2 H), 3.76 (s, 3 H), 3.47 - 3.39 (m, 2 H), 2.75 (s, 3 H), 2.41 (s, 3 H), 0.87 - 0.83 (m, 2 H), 0.09 (s, 9 H).

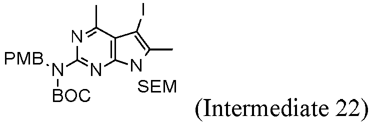

### Intermediate 22: tert-butyl N-(5-iodo-4,6-dimethyl-7-SEM-pyrrolo[2,3-d]pyrimidin-2-yl)-N-[(4-methoxyphenyl)methyl]carbamate

To a solution of **Intermediate 17** (150 g, 278 mmol, 1.00 eq) and Boc₂O (72.9 g, 334 mmol, 76.7 mL, 1.20 eq) in THF (1.50 L) was cooled to -30°C under N₂, LiHMDS (1 M, 278 mL, 1.00 eq) was added to the mixture slowly, the mixture was stirred at -30°C ~ - 10°C for 1 h. TLC (petroleum ether:EtOAc 5:1, product Rf 0.6) showed the reaction was completed. The reaction mixture was added into NH₄Cl(aq) (300 mL). The resulting mixture was extracted with EtOAc (300 mL × 3). The combined organic layers were dried over Na₂SO₄, filtered. No further purification. tert-butyl N-(5-iodo-4,6-dimethyl-7-SEM-pyrrolo[2,3-d]pyrimidin-2-yl)-N-[(4-methoxyphenyl)methyl]carbamate **(Intermediate 22)** (400 g, crude) was obtained as yellow oil.

¹H-NMR (400 MHz, CDCl₃): 7.32 - 7.29 (m, 2H), 6.79 - 6.77 (m, 2H), 5.55 (s, 2H), 5.05 (s, 2H), 3.76 (s, 3H), 3.49 - 3.45 (m, 2H), 2.92 (s, 3H), 2.51 (s, 3H), 1.43 (s, 9H), 0.88 - 0.84 (m, 2H), -0.06 (m, 9H)

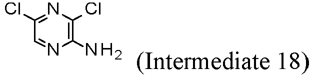

To a mixture of 3-chloropyrazin-2-amine (200 g, 1.54 mol, 1.00 eq) in CHCl₃ (2.00 L) was added NCS (247 g, 1.85 mol, 1.20 eq) in one portion at 25°C under N₂. The mixture was heated to 70°C for 12 h. TLC (petroleum ether:EtOAc 3:1) indicated consumption of starting material and one new spot (Rf 0.5) formed. The mixture was cooled to 20°C. The residue was poured into water (2.00 L) and extracted with EtOAc (500 mL × 4). The combined organic phase was washed with brine (500 mL × 2), dried with Na₂SO₄, filtered and concentrated in vacuum. The crude product was triturated with petroleum ether:EtOAc (petroleum ether:EtOAc 12:1) at 20°C for 3 h, filtered and concentrated in vacuum. 3,5-dichloropyrazin-2-amine (Intermediate 18) (534 g, 2.87 mol, 92.9% yield, 88% purity) was obtained as yellow solid.

LCMS: Rt 0.574 min, m/z 164 [M+1]+.

¹H-NMR (400 MHz, CDCl₃): 7.96 (s, 1H), 5.21 - 4.99 (br, 2H).

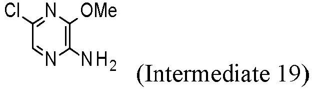

### Intermediate 19: 5-chloro-3-methoxy-pyrazin-2-amine

To a mixture of compound **Intermediate 18** (190 g, 1.02 mol, 1.00 eq) in MeOH (1.50 L) was added NaOMe (367 g, 2.04 mol, 30% purity, 2.00 eq) in one portion at 20°C under N₂. The mixture was heated to 70°C and stirred for 4 h. TLC (petroleum ether:EtOAc 3:1) indicated complete consumption of starting material and one new spot (Rf 0.4) formed. The mixture was cooled to 20°C. The residue was poured into water (2.00 L) and extracted with EtOAc (600 mL × 4). The combined organic phase was washed with brine (500 mL × 2), dried with Na₂SO₄, filtered and concentrated in vacuum. The crude product was triturated (petroleum ether:EtOAc 7:1, 300 mL) at 20°C for 1 h, filtered and concentrated in vacuum to give 5-chloro-3-methoxy-pyrazin-2-amine **(Intermediate 19)** (348 g, 2.01 mol, 98.3% yield, 92% purity) as yellow solid.

LCMS: Rt 0.474 min, m/z 160 [M+1]+.

¹H-NMR (400 MHz, CDCl₃): 7.61 (s, 1H), 4.91 (br, 2H), 4.08 (s, 3H)

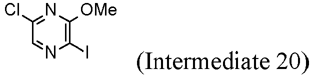

### Intermediate 20: 5-chloro-2-iodo-3-methoxy-pyrazine

To a mixture of compound **Intermediate 19** (100 g, 607 mmol, 1.00 eq), CH₂I₂ (243 g, 910 mmol, 73.4 mL, 1.50 eq) and CuI (11.5 g, 60.7 mmol, 0.10 eq) in THF (600 mL) was added isoamyl nitrite (284 g, 2.43 mol, 327 mL, 4.00 eq) in one portion at 20°C under N₂. The mixture was stirred at 65°C for 3 h. TLC (petroleum ether:EtOAc 3:1) indicated complete consumption of starting material and one new spot (Rf 0.70) formed. The mixture was cooled to 20°C. The residue was poured into water (1.00 L) and extracted with EtOAc (400 mL × 4). The combined organic phase was washed with brine (300 mL × 2), dried with Na₂SO₄, filtered and concentrated under reduced pressure to give a residue. The residue was purified by silica gel chromatography (column height: 300 mm, diameter: 100 mm, 100-200 mesh silica gel, petroleum ether:EtOAc 1:0 to 100:1) to give compound 5-chloro-2-iodo-3-methoxy-pyrazine **(Intermediate 20)** (114 g, 382 mmol, 62.9% yield, 90.7% purity) as yellow solid.

LCMS: Rt 0.908 min, m/z 267 [M+1]+.

¹H-NMR (400 MHz, CDCl₃): 8.00 (s, 1H), 4.11 (s, 3H)

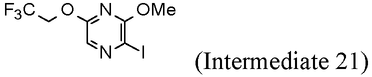

### Intermediate 21: 2-iodo-3-methoxy-5-(2,2,2-trifluoroethoxy)pyrazine

To a mixture of **Intermediate 20** (88.0 g, 305 mmol, 1.00 eq) and 2,2,2-trifluoroethanol (45.8 g, 458 mmol, 32.9 mL, 1.50 eq) in THF (500 mL) at 0°C ~ 10°C under N₂, then t-BuOK (34.2 g, 305 mmol, 1.00 eq) was added portion wise. The mixture was stirred at 20°C for 24 h. TLC (petroleum ether:EtOAc 10:1, **Intermediate 20** Rf 0.7) showed the remaining starting material, two spots (main spot Rf 0.5) were formed, and desired mass was detected by LCMS. The residue was partitioned between EtOAc (300 mL × 3) and water (500 mL). The combined organic layers were washed with brine (200 mL × 2). The organic phase was separated, dried with Na₂SO₄, filtered and concentrated under reduced pressure to give a residue. The residue was purified by silica gel chromatography (column height: 500 mm, diameter: 120 mm, 100-200 mesh silica gel, petroleum ether:EtOAc 1:0 to 100:1) to give 2-iodo-3-methoxy-5-(2,2,2-trifluoroethoxy)pyrazine **(Intermediate 21)** (54.0 g, 145 mmol, 23.8% yield, 90% purity) as yellow solid.

LCMS: Rt 0.963 min, m/z 335 [M+1]+.

¹H-NMR (400 MHz, CDCl₃): 7.80 (s, 1H), 4.73 (m, 2H), 4.02 (s, 3H)

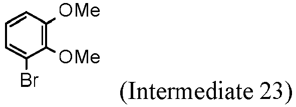

### Intermediate 23: 1-bromo-2,3-dimethoxy-benzene

To a solution of TMEDA (404 g, 3.48 mol, 525 mL, 1.20 eq) in THF (2.00 L), N-butyllithium (2.50 M, 1.40 L, 1.21 eq) was dropped wise added over 1hr at -20- -5°C under nitrogen atmosphere, then 1,2-dimethoxybenzene (400 g, 2.90 mol, 370.37 mL, 1 eq) was dropped wise added into the mixture over 1hr at -20 - 0°C, and Br₂ (500 g, 3.13 mol, 161.29 mL, 1.08 eq) was dropped wise added into the mixture over 2 h at -70 to - 65°C under nitrogen atmosphere. It was kept stirring for 1 h at 0°C. TLC (petroleum ether:EtOAc 20:1) showed remaining starting material and a new spot (Rf 0.50) was formed. The mixture was poured into Na₂SO₃(aq) solution (1000 mL) under adequate stirring, extracted with MTBE (500 mL × 3), the combined organic phase was washed with brine (500 mL × 3), dried with Na₂SO₄, then filtered, the filtrate was concentrated under reduced pressure to give brown liquid. The residue was purified by silica gel chromatography (petroleum ether:EtOAc 100:1, Rf 0.4). The product 1-bromo-2,3-dimethoxy-benzene **(Intermediate 23)** (740 g, 3.41 mol, 58.9% yield) was obtained as a colourless liquid.

¹H-NMR (400 MHz, CDCl₃): 7.04 (dd, J 8.01, 1.41 Hz, 1H), 6.80 - 6.88 (m, 1H), 6.74 - 6.79 (m, 1H), 3.77 (d, J 1.34 Hz, 6H)

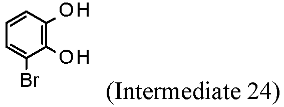

### Intermediate 24: 3-bromobenzene-1,2-diol

To a solution of compound **Intermediate 23** (400 g, 1.84 mol, 1.00 eq) in DCM (600 mL) was added a solution of BBr₃ (808 g, 3.23 mol, 311 mL, 1.75 eq) in DCM (1000 mL) at 0°C. After addition, the reaction mixture was stirred at 25°C for 2 h. TLC (petroleum ether:EtOAc 5:1) indicated complete consumption of starting material and one new spot formed. The reaction was quenched with MeOH (800 mL) at 0°C. Then poured into H₂O (1000 mL) and extracted with DCM (500 mL × 4). The combined organic layer was washed with brine (1000 mL), dried over Na₂SO₄ and concentrated to give the crude product. The crude product was used directly without purification for next step. 3-bromobenzene-1,2-diol **(Intermediate 24)** (280 g, 1.48 mol, 80.4% yield) was obtained as a brown liquid.

¹H-NMR (400 MHz, CDCl₃): 6.92 (d, J 8.07 Hz, 1H), 6.79 (d, J 7.95 Hz, 1H), 6.79 (d, J 7.95 Hz, 1H), 5.69 (s, 1H), 5.56 (s, 1H)

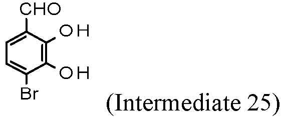

### Intermediate 25: 4-bromo-2,3-dihydroxy-benzaldehyde

**Intermediate 24** (150 g, 794 mmol, 1.00 eq) was dissolved in ACN (3.00 L), MgCl₂ (453.37 g, 4.76 mol, 195.42 mL, 6 eq), Et₃N (642.45 g, 6.35 mol, 883.70 mL, 8 eq), HCHO (167 g, 5.56 mol, 153 mL, 7.00 eq) and DMAP (9.70 g, 79.4 mmol, 0.1 eq) was added, the mixture was heated to 60-70°C and stirred at 60-70°C for 12 h under nitrogen atmosphere. LCMS showed 17% starting material remaining. Two equal batches were combined and the pH of the mixture was adjusted to 3.0 with 1 M HCl (aq) (10.0 L), then was extracted with EtOAc (4.00 L × 2), the organic phase was combined and washed with brine (2.00 L × 2), dried over Na₂SO₄ and concentrated in vacuum to give a residue. The residue was suspended in petroleum (600 mL) and EtOAc (200 mL), the mixture was heated to 55°C and stirred for 1 h, then was cooled to 20°C and stirred for 1 h. The solid was collected by filtration and dried in vacuum. 4-bromo-2,3-dihydroxy-benzaldehyde **(Intermediate** 25) (180 g, 829 mmol, 52.3% yield) was obtained as a yellow solid.

¹H-NMR (400 MHz, DMSO-d6): 10.12 (d, J 3.55 Hz, 1H), 7.09 - 7.39 (m, 2H)

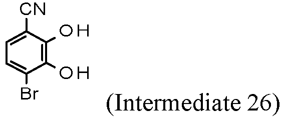

### Intermediate 26: 4-bromo-2,3-dihydroxy-benzonitrile

**Intermediate 25** (300 g, 1.38 mol, 1.00 eq) was suspended in H₂O (3.00 L) and CH₃COOH (83.0 g, 1.38 mol, 79.1 mL, 1.00 eq), sulfamic acid (313 g, 2.76 mol, 2.00 eq) was added portion wise and the mixture was heated to 50°C and stirred for 3 h. TLC (petroleum ether:EtOAc 1:1, Rf 0.2) showed compound 4 was consumed completely. The pH of the mixture was adjusted to 3.0 with NaHCO₃, the mixture was extracted with EtOAc (2.00 L × 3), the organic phase was combined and washed with brine(2.00 L × 2), dried over Na₂SO₄ and concentrated in vacuum to give the crude product. The crude product was suspended in petroleum (600 mL) and stirred at 30°C for 30 min, the solid was collected by filtration and dried in vacuum. 4-bromo-2,3-dihydroxy-benzonitrile **(Intermediate 26)** (261 g, 1.22 mol, 88.2% yield) was obtained as a brown solid.

¹H-NMR (400 MHz, DMSO-d6): 7.07 - 7.15 (m, 1H), 7.00 - 7.07 (m, 1H)

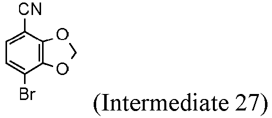

### Intermediate 27: 7-bromo-1,3-benzodioxole-4-carbonitrile

**Intermediate 26** (261 g, 1.22 mol, 1.00 eq) was dissolved in DMF (1.50 L), CH₂I₂ (490 g, 1.83 mol, 148 mL, 1.50 eq) and K₂CO₃ (506 g, 3.66 mol, 3.00 eq) was added, the mixture was heated to 60°C and stirred for 12 h under nitrogen atmosphere. TLC (petroleum ether:EtOAc 1:1, Rf 0.7) showed the reaction was completed. The mixture was concentrated in vacuum and the residue was added EtOAc (2.00 L) and water (2.00 L), the mixture was filtered and the filtration was separated and the aqueous phase was extracted with EtOAc(1.00 L × 3), the organic phase was combined and washed with brine (2.00 L × 3), dried over Na₂SO₄ and concentrated in vacuum. The residue was suspended in MTBE (400 mL) and stirred at 30°C for 1 h, the solid was collected by filtration and dried in vacuum. 7-bromo-1,3-benzodioxole-4-carbonitrile **(Intermediate** 27) (150 g, 664 mmol, 54.4% yield) was obtained as a brown solid.

¹H-NMR (400 MHz, DMSO-d6): 7.21 - 7.16 (m, 2H), 6.33 (s, 2H)

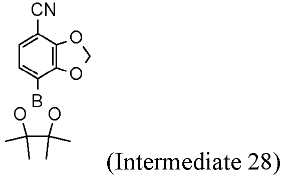

### Intermediate 28: 7-(4,4,5,5-tetramethyl-1,3,2-dioxaborolan-2-yl)-1,3-benzodioxole-4-carbonitrile

**Intermediate 27** (128 g, 566 mmol, 1.00 eq) was dissolved in 1,4-dioxane (1.25 L), KOAc (167 g, 1.70 mol, 3.00 eq) and Pin₂B₂ (216 g, 849 mmol, 1.50 eq) was added, the mixture was degassed with nitrogen in vacuum cycles for three times and Pd(dppf)Cl₂ (20.7 g, 28.3 mmol, 0.05 eq) was added, the mixture was degassed with nitrogen in vacuum cycles for three times and was heated to 100°C and stirred for 12 h. TLC (petroleum ether:EtOAc 1:1, Rf 0.6) showed the reaction was complete. The mixture was cooled to 30°C and filtered, the filtration was concentrated in vacuum. The residue was purified by silica gel chromatography (petroleum ether:EtOAc 10:1 to 1:1, Rf 0.6). 7-(4,4,5,5-tetramethyl-1,3,2-dioxaborolan-2-yl)-1,3-benzodioxole-4-carbonitrile **(Intermediate** 28) (130 g, 476 mmol, 84.1% yield) was obtained as an off-white solid, which was confirmed by ¹H NMR. ¹H-NMR (400 MHz, DMSO-d6): 7.09 - 7.17 (m, 2H), 6.26 (s, 2 H), 1.29 (s, 12H)

### Examples:

### Preparation of 4-(2-amino-4,6-dimethyl-7H-pyrrolo[2,3-d]pyrimidin-5-yl)-3-methoxybenzonitrile (C1)

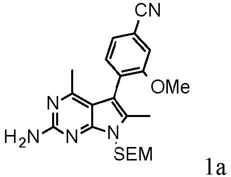

### 4-(2-amino-4,6-dimethyl-7-SEM-7H-pyrrolo[2,3-d]pyrimidin-5-yl)-3-methoxybenzonitrile (1a)

To a 1 L round bottom flask was added **Intermediate** 8 (22.7 g, 54.3 mmol), **Intermediate 9** (17.6 g, 67.8mmol) and potassium carbonate (22.5 g, 162.8 mmol). After purging with nitrogen, dioxane (400 ml) and water (40 ml) were added and the solution was bubbled with nitrogen for ~10 min, while stirring, before addition of Pd(PPh₃)₄ (6.27 g, 5.43 mmol). After heating at 100°C overnight, the reaction was concentrated to about half the volume, diluted with water (100 ml), then extracted with EtOAc (200 ml, 2x50 ml). The organic layers were combined, washed with sat. aqueous NaCl (2x100 ml), dried over MgSO₄, filtered and concentrated to about 50 ml. The residue was diluted with hexane (50 ml), poured onto a 9x9 cm hexane-wet silica plug and eluted with 0:100 to 50:50 hexane:EtOAc (~200 ml fractions). Fractions containing product (by TLC) were evaporated to give an oil which solidified when drying under high vacuum to give 4-(2-amino-4,6-dimethyl-7-SEM-7H-pyrrolo[2,3-d]pyrimidin-5-yl)-3-methoxybenzonitrile **(1a)** (24.14 g, 104%) as a light brown solid (contains ~11% triphenylphosphine oxide by LCMS).

LCMS: Rt 3.92 min, m/z: [M+H]+ Calcd for C₂₂H₂₉N₅O₂Si 424.22; Found 424.20.

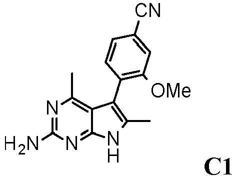

### 4-(2-amino-4,6-dimethyl-7H-pyrrolo[2,3-d]pyrimidin-5-yl)-3-methoxybenzonitrile (C1)

To a 1 L round bottom flask was added **1a** (24.14 g, 57.00 mmol), DCM (250 ml) and trifluoroacetic acid (60 ml). After stirring at room temp for 7 h, the solvent was evaporated. To the resulting residue was added dioxane (250 ml) and ethylenediamine (25 ml), which was stirred at room temperature overnight. The solvent was evaporated, and the residue taken up in EtOAc (250 ml), washed with water (100 ml), sat. aqueous NaCl (100 ml), dried over MgSO₄, filtered and concentrated to ~100 ml. The resulting slurry was poured onto a 9x9 cm EtOAc-wet silica plug and eluted with 0:100 to 10:90 MeOH:EtOAc (~200 ml fractions). Fractions containing product (by TLC) were evaporated to give 4-(2-amino-4,6-dimethyl-7H-pyrrolo[2,3-d]pyrimidin-5-yl)-3-methoxybenzonitrile (**C1**) (11.92 g, 71%) as a white solid.

LCMS: Rt 2.76 min, m/z: [M+H]+ Calcd for C₁₆H₁₅N₅O 294.13; Found 294.10.

¹H-NMR (400 MHz, DMSO-d6): 1.97 (s, 3H, Ar-CH₃), 2.07 (s, 3H, Ar-CH₃), 3.78 (s, 3H, O-CH₃), 5.90 (s, 2H, NH₂), 7.38 (d, 1H, ArH, J 7.6 Hz), 7.46 (dd, 1H, ArH, J 7.6 Hz, 1.2Hz), 7.53 (d, 1H, ArH, J 1.2 Hz), 11.16 (s, 1H, NH).

¹³C{¹H}-NMR (100 MHz, DMSO-*d₆*,): 12.0, 21.3, 56.1, 107.0, 110.3, 111.0, 114.6, 119.5, 124.7, 129.9, 130.7, 133.6, 153.7, 158.19, 158.22, 159.5.

### Preparation of 5-(7-fluoro-1,3-benzodioxol-4-yl)-4,6-dimethyl-7H-pyrrolo[2,3-d]pyrimidin-2-amine (C2)

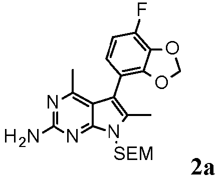

### 5-(7-fluoro-1,3-benzodioxol-4-yl)-7-SEM-4,6-dimethyl-7H-pyrrolo[2,3-d]pyrimidin-2-amine (2a)

To a 1 L round bottom flask was added **Intermediate 8** (8.0 g, 19.12 mmol), **Intermediate 12** (6.11 g, 22.95 mmol) and potassium carbonate (7.93 g, 57.37 mmol). After purging with nitrogen, dioxane (100 ml) and water (20 ml) were added then the solution was bubbled with nitrogen for ~10 min before addition of Pd(PPh₃)₄ (0.66 g, 0.57 mmol). After heating at 100°C for overnight, the reaction was concentrated to about half the volume then partitioned between EtOAc (100 ml) and water (100 ml). The organic layer was washed with saturated NaCl (50 ml), dried over MgSO₄, filtered, evaporated on silica (~15 g) then subjected to flash chromatography with 0:100 to 50:50 EtOAc:hexane (80 g silica). Fractions containing product (TLC) were evaporated to give 5-(7-fluoro-1,3-benzodioxol-4-yl)-7-SEM-4,6-dimethyl-7H-pyrrolo[2,3-d]pyrimidin-2-amine (2a) (7.68 g, 93%) as a pale-yellow oil.

LCMS: Rt 2.83 min, m/z: [M+H]+ Calcd for C21H27FN4O3Si 431.19; Found 431.10.

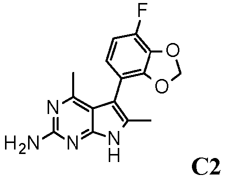

### 5-(7-fluoro-1,3-benzodioxol-4-yl)-4,6-dimethyl-7H-pyrrolo[2,3-d]pyrimidin-2-amine (C2)

To a 1 L round bottom flask was added 2a (7.68 g, 17.84 mmol), DCM (80 ml) and trifluoroacetic acid (25 ml). After stirring at room temp for 6 h, the solvent was evaporated. To the resulting residue was added dioxane (80 ml) and ethylenediamine (20 ml), which was stirred at room temperature overnight. The solvent was then evaporated and the residue taken up in EtOAc (150 ml), washed with water (50 ml), saturated NaCl (50 ml), dried over MgSO₄, filtered, evaporated on silica (~15 g) then subjected to flash chromatography with 0:100 to 50:50 EtOAc:hexane (80 g silica). Fractions containing product (TLC) were evaporated to give 5-(7-fluoro-1,3-benzodioxol-4-yl)-4,6-dimethyl-7H-pyrrolo[2,3-d]pyrimidin-2-amine **(C2)** (3.20 g, 59%) as an off-white solid.

LCMS: Rt 2.22 min, m/z: [M+H]+ Calcd for C15H13FN4O2 301.11; Found 301.10.

¹H-NMR (400 MHz, DMSO-d6): 2.13 (s, 6H, Ar-CH3), 5.91 (s, 2H, NH2), 6.11 (d, 1H, CH2, J 7.6 Hz), 6.80 (dd, 1H, ArH, J 8.8 Hz, 5.2Hz), 6.89 (dd, 1H, ArH, J 10.0Hz, 8.8 Hz), 11.16 (s, 1H, NH).

### Preparation of 5-[3-methoxy-5-(2,2,2-trifluoroethoxy)pyrazin-2-yl]-4,6-dimethyl-7H-pyrrolo[2,3-d]pyrimidin-2-amine (C3)

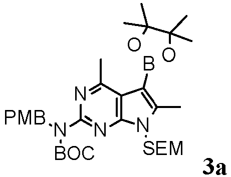

### tert-Butyl N-[4,6-dimethyl-5-(4,4,5,5-tetramethyl-1,3,2-dioxaborolan-2-yl)-7-SEM-pyrrolo[2,3-d]pyrimidin-2-yl]-N-[(4-methoxyphenyl)methyl]carbamate (3a)

To a solution of **Intermediate 22** (200 g, 313 mmol, 1.00 eq) and 2-isopropoxy-4,4,5,5-tetramethyl-1,3,2-dioxaborolane (78.6 g, 422 mmol, 86.2 mL, 1.35 eq) in THF (2.00 L) was cooled to -15°C under N₂, i-PrMgCl-LiCl (1.3 M, 361 mL, 1.50 eq) was added to the mixture slowly, the mixture was stirred at -15°C ~ -10°C for 1 h. TLC (petroleum ether:EtOAc 5:1, product Rf 0.3) showed the starting material was consumed completely. The reaction mixture was added into saturated aqueous NH₄Cl (1.00 L). The resulting mixture was extracted with EtOAc (500 mL × 3). The combined organic layers were dried over Na₂SO₄, filtered and concentrated on vacuum. The residue was purified by silica gel chromatography (column height: 500 mm, diameter: 120 mm, 100 - 200 mesh silica gel, petroleum ether:EtOAc 1:0 to 10:1) to give compound tert-butyl N-[4,6-dimethyl-5-(4,4,5,5-tetramethyl-1,3,2-dioxaborolan-2-yl)-7-SEM-pyrrolo[2,3-d]pyrimidin-2-yl]-N-[(4-methoxyphenyl)methyl]carbamate **(3a)** (130 g, 193 mmol, 61.7% yield, 95% purity) as yellow oil.

LCMS: Rt 1.091 min, m/z 639 [M+1]+.

¹H-NMR (400 MHz, CDCl₃): 7.42 - 7.34 (m, 2H), 6.87 - 6.82 (m, 2H), 5.65 (s, 2H), 5.14 (s, 2H), 3.85 (s, 3H), 3.58 - 3.51 (m, 2H), 2.95 (s, 3H), 2.77 (s, 3H), 1.50 (s, 9H), 1.45 (s, 12H), 0.96 - 0.87 (m, 2H), 0.00 (m, 9H).

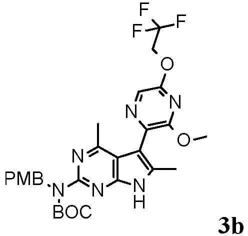

### tert-Butyl N-[(4-methoxyphenyl)methyl]-N-[5-[3-methoxy-5-(2,2,2-trifluoroethoxy)pyrazin-2-yl]-4,6-dimethyl-7-SEM-pyrrolo[2,3-d]pyrimidin-2-yl]carbamate (3b)

A mixture of compound 3a (92.9 g, 145 mmol, 1.00 eq), **Intermediate 21** (54.0 g, 145 mmol, 1.00 eq), Pd(dppf)Cl₂.CH₂Cl₂ (11.8 g, 14.5 mmol, 0.10 eq), K₃PO₄ (92.6 g, 436 mmol, 3.00 eq) and H₂O (141 g, 7.86 mol, 141 mL, 54.0 eq) in dioxane (700 mL) was degassed and purged with N₂ for 3 times, and then the mixture was stirred at 110°C for 14 h under N₂ atmosphere. TLC (petroleum ether:EtOAc 3:1, product Rf 0.3) showed the starting material was consumed completely. The mixture was added water (800 mL), extracted with EtOAc (500 mL × 3), washed with brine (300 mL × 2), dried over Na₂SO₄, filtered and concentrated on vacuum. The residue was purified by silica gel chromatography (column height: 400 mm, diameter: 100 mm, 100 - 200 mesh silica gel, petroleum ether:EtOAc 1:0 to 10:1) to give tert-butyl N-[(4-methoxyphenyl)methyl]-N-[5-[3-methoxy-5-(2,2,2-trifluoroethoxy)pyrazin-2-yl]-4,6-dimethyl-7-SEM-pyrrolo[2,3-d]pyrimidin-2-yl]carbamate **(3b)** (37.0 g, 48.1 mmol, 33.0% yield, 93.5% purity) as yellow oil.

LCMS: Rt 1.103 min, m/z 719 [M+1]+.

¹H-NMR (400 MHz, CDCl₃): 8.08 - 7.95 (s, 1H), 7.34 - 7.29 (m, 2H), 6.80 - 6.76 (m, 2H), 5.60 (s, 2H), 5.07 (s, 2H), 4.89 - 4.76 (m, 2H), 3.95 (s, 3H), 3.76 (s, 3H), 3.58 - 3.49 (m, 2H), 2.37 (s, 3H), 2.31 (s, 3H), 1.43 (s, 9H), 0.91 - 0.87 (m, 2H), -0.04 (s, 9H).

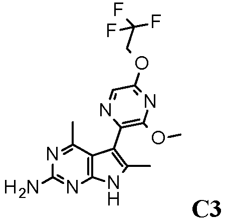

### 5-[3-methoxy-5-(2,2,2-trifluoroethoxy)pyrazin-2-yl]-4,6-dimethyl-7H-pyrrolo[2,3-d]pyrimidin-2-amine (C3)

A mixture of compound **3b** (37.0 g, 47.8 mmol, 1.00 eq), TFA (286 g, 2.51 mol, 186 mL, 52.4 eq) and DCM (100 mL) was stirred at 40°C for 12 h. The reaction solution was concentrated on vacuum. The residue was added to NH₃(aq) (84.6 g, 724 mmol, 93.0 mL, 15.1 eq) and stirred at 100°C for 2 h. LCMS showed that **3b** was completely consumed and the desired mass was detected. The mixture was filtered and the solid concentrated in vacuum. The solid was dissolved in DCM (150 mL) and aqueous phase removed. The combined organic phase was dried over Na₂SO₄, filtered and concentrated in vacuum. The residue was purified by prep-HPLC (column: Phenomenex Synergi Max-RP 250 × 50mm × 10 um; mobile phase: [water (0.1% TFA)-ACN]; B%:21%-37%, 15min). 5-[3-methoxy-5-(2,2,2-trifluoroethoxy)pyrazin-2-yl]-4,6-dimethyl-7H-pyrrolo[2,3-d]pyrimidin-2-amine trifluoracetic acid salt **(C3 TFA salt)** (13.5 g, 36.2 mmol, 75.8% yield, 99% purity, TFA salt) was obtained as off-white solid.

LCMS: Rt 0.778 min

Combined batches of **C3 TFA salt** was dissolved in DCM (4.00 L) and pH adjusted to 8 ~ 9 by sat. NaHCO₃ solution. The combined organic phase was washed with brine (100 mL × 2), dried with Na₂SO₄, filtered and concentrated in vacuum. Compound 5-[3-methoxy-5-(2,2,2-trifluoroethoxy)pyrazin-2-yl]-4,6-dimethyl-7H-pyrrolo[2,3-d]pyrimidin-2-amine (C3) (28.3 g, 76.9 mmol, 83.2% yield, 100% purity) was obtained as off-white solid.
LCMS: Rt 0.778 min, m/z 369 [M+1]+.
¹H-NMR (400 MHz, DMSO-d6): 11.20 (br, 1H), 8.08 (s, 1H), 5.91 (br, 2H), 5.11 (m, 2H), 3.91 (s, 3H), 2.09 (s, 3H), 2.01 (s, 3H)

### Preparation of 7-(2-amino-4,6-dimethyl-7H-pyrrolo[2,3-d]pyrimidin-5-yl)-1,3-benzodioxole-4-carbonitrile (C4)

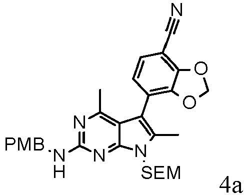

### 7-[2-[(4-methoxyphenyl)methylamino]-4,6-dimethyl-7H-pyrrolo[2,3-d]pyrimidin-5-yl]-1,3-benzodioxole-4-carbonitrile (4a)

Two equal batches containing **Intermediate 28** (65.0 g, 238 mmol, 1.10 eq) and **Intermediate 17** (118 g, 216 mmol, 1.00 eq) was dissolved in DMSO (650 mL), Na₂CO₃ (72.2 g, 682 mmol, 3.15 eq) was added, the mixture was degassed with nitrogen in vacuum cycles for three times and Pd(dppf)Cl₂ (7.92 g, 10.8 mmol, 0.05 eq) was added, the mixture was degassed with nitrogen in vacuum cycles for three times and was heated to 100°C and stirred for 12 h under nitrogen atmosphere. LCMS showed the reaction was complete and the desired mass was detected, and the mixture was cooled to 40°C. The two equal batches were combined and filtered, the filtration was added water (1.50 L) and extracted with EtOAc (1.00 L × 4), the organic phase was combined and washed with brine (2.00 L × 3), dried over Na₂SO₄ and concentrated in vacuum to give a residue. The residue was purified by column chromatography (silica, petroleum ether:EtOAc 10:1 to 5:1, Rf 0.5). 7-[2-[(4-methoxyphenyl)-methylamino]-4,6-dimethyl-7H-pyrrolo[2,3-d]pyrimidin-5-yl]-1,3-benzodioxole-4-carbonitrile **(4a)** (215 g, 377 mmol, 87.2% yield, 97.9% purity) was obtained as a brown oil.
LCMS: Rt 0.982 min m/z 558.6 [M+1]+
¹H-NMR (400 MHz, CDCl₃): 7.32 - 7.39 (m, 1H), 7.28 - 7.40 (m, 2H), 7.15 (d, J 8.19 Hz, 1H), 6.91 (dd, J 8.38, 4.83 Hz, 3H), 6.18 (d, J 12.47 Hz, 2H), 5.48 - 5.68 (m, 2H), 5.32 (br, 1H), 4.65 (d, J 5.87 Hz, 2H), 4.18 (d, J 7.09 Hz, 1H), 3.85 (s, 3H), 3.61 (t, J 8.07 Hz, 2H), 2.33 (d, J 10.88 Hz, 6H), 0.86 - 1.01 (m, 2H), 0.00 (s, 9H)

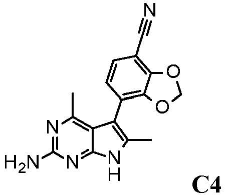

### 7-(2-amino-4,6-dimethyl-7H-pyrrolo[2,3-d]pyrimidin-5-yl)-1,3-benzodioxole-4-carbonitrile (C4)

Compound **4a** (100 g, 179 mmol, 1.00 eq) was dissolved in TFA (481 g, 4.22 mol, 312 mL, 23.5 eq), the mixture was stirred at 0 - 10°C under nitrogen atmosphere for 12 h. The mixture was concentrated in vacuum at 35°C and the residue was dissolved in THF (600 mL) and NH₃.H₂O (186 g, 1.33 mol, 204 mL, 25.0% purity, 7.40 eq) was added, the mixture was stirred at 10-20°C for 12 h. TLC (DCM:MeOH 10:1, Rf 0.4) showed the reaction was complete. The mixture was combined with another equal batch and concentrated in vacuum. The residue was purified by prep-HPLC (neutral condition, column: Phenomenex Luna C18 250 × 50 mm × 10 µm; mobile phase: Water-ACN; B%: 30.0%-47.8%,17 min), the product was suspended in saturated NaHCO₃ solution (500 mL) and DCM (4.00 L), and stirred at 20 - 25°C for 1 h, the solid was collected by filtration and washed with distilled water (200 mL × 2) and dried in vacuum. 7-(2-amino-4,6-dimethyl-7H-pyrrolo[2,3-d]pyrimidin-5-yl)-1,3-benzodioxole-4-carbonitrile (C4) (34.0 g, 110 mmol, 30.7% yield, 99.6% purity) was obtained as an off-white solid.
LCMS: Rt 0.742 min m/z 308.2 [M+1]+
HPLC: Rt 2.320 min, 99.7% purity
¹H-NMR (400 MHz, DMSO-d6): 11.32 (s, 1H), 7.25 (d, J 8.28 Hz, 1H), 6.96 (d, J 8.28 Hz, 1H), 6.24 (br, 2H), 6.00 (s, 2H), 2.16 (d, J 3.51 Hz, 6H)

### Biological activity

Testing of compounds in *in vitro* and *in vivo* model for anti-leishmanial activity are well established, especially for VL. Effects observed *in vitro* for current drug treatments translates well to *in vitro* efficacy in animal models of VL who may be used to understand the potential to achieve a beneficial effect in infected patients.

Experimental *in vitro* models for Leishmaniasis are well described. For example, mouse macrophages infected with spleen derived *L. infantum* or *L. donovani* amastigotes obtained from heavily infected donor hamsters may be used to investigate anti-parasite efficacy of investigational VL treatments. The VL drug miltefosine display an *in vitro* anti-parasitic efficacy for *L. infantum and L. donovani* infected macrophages of IC₅₀ 3.50 ± 0.40 and 1.80 ± 0.40, respectively. [IJP: Drugs and Drug Resistance, 2018, 8, Issue 1, 81-86] The *in vitro* efficacy of compounds developed for treatment of CL may be investigated in a similar manner using CL associated *Leishmania* strains.

*In vivo* models of VL allowing for efficacy testing novel treatments are well developed, in particular mouse and hamster models for VL are frequently being used to assess treatment efficacy of novel anti-infectives. However, these *in vivo* models differ in how well they reflect the different known leishmaniasis clinical manifestations (that may vary from a localized cutaneous ulcer to skin and mucosa metastatic lesions, or to the colonization of internal organs such as the spleen, liver, and bone marrow) associated with different pathological mechanisms.

Inbred mouse strains such as BALB/c and C57BL/6 mice strains, are susceptible for infection by *L. donovani* and *L. infantum* and may thus be used as models to study the acute phase increase of the parasite burden. However, as the infected mouse over the course of 4-8 weeks is able to mount an anti-leishmanial cellular immune response and control the infection mouse models are generally less suited to study effects in the chronic phase of the infection.

It has been shown that many hamster species are susceptible to *L. donovani* infection, the Syrian golden hamster *(Mesocricetus auratus)* establishes a good model for VL and provides a more synchronous infection in liver and spleen that can develop into chronic infection more similar to human VL manifestations. Systemic infection of the hamster with *L. donovani* results in a relentless increase in visceral parasite burden, progressive cachexia, hepatosplenomegaly, pancytopenia, hypergammaglobulinemia, and ultimately death.

There are currently no validated CL models available to predict the clinical efficacy of novel CL treatments.

### In vitro testing

### Intracellular amastigote susceptibility assay

To obtain primary peritoneal macrophages, Swiss mice were stimulated by intraperitoneal injection of 1 mL 2% starch in PBS 48 h prior to cell collection. Animals were euthanized with a CO2 overdose and upon removal of the skin, 10 mL of RPMI-1640 (Life Technologies) was injected into the peritoneal cavity to collect the macrophages that were then seeded into 96-well plates at a final concentration of 30,000 cells/well in 100 µL of RPMI-1640 macrophage medium, supplemented with 5% iFCS, 2% penicillin/streptomycin and 1% L-glutamine (Life technologies). After 24 h, the cells were infected with spleen derived *L. infantum* (MHOM/MA(BE)/67/ITMAP263) *or L. donovani* (MHOM/ET/67/L82) amastigotes obtained from heavily infected donor hamsters. The amastigotes were purified using two centrifugation steps and diluted to obtain an infection ratio of 5:1 in RPMI-1640 cell culture medium. After incubation for 2 h, compound dilutions were added. Drug activity was evaluated after a 96-h incubation period without washing of the cells and without renewal of the drugged culture medium. Cells were stained with Giemsa for microscopic evaluation of cellular amastigote burdens. Percentage reduction compared to the burdens in the infected non-treated control wells was used as a measure for drug activity.

### In vitro cytotoxicity assay on MRC-5 cells

MRC-5SV2 cells were cultured in MEM with Earl's salts-medium (Life Technologies) supplemented with L-glutamine, NaHCO3 and 5% inactivated fetal bovine serum (iFCS, Life Technologies). For the cytotoxicity assay, the cells were seeded at a concentration of 15,000 cells/well and 4-fold compound dilutions were added with a highest in-test concentration of 200 µM. After 3 days incubation at 37°C and 5% CO2, resazurin (Sigma Aldrich, Diegem, Belgium) was added for fluorescence reading (Tecan^{®}, GENios) after another 4 h of incubation. Cell viability was compared to the untreated control wells and the cytotoxic concentration 50% (CC₅₀) was calculated of each compound.

Anti-leishmanial efficacy data for *L. infantum* and *L. donovani* generated for representative compounds are provided in Table 1 and 2 referenced against miltefosine.

**Table 1. Results, compounds tested for L. infantum (MHOM/MA/67/ITMAP263) activity**

| | | | | |
|---|---|---|---|---|
| | | | | |

| Compound | IC₅₀ (µM) | IC₉₀ (µM) | SI | n |
|---|---|---|---|---|
| (C4) | 0.15 | 0.37 | >417 | 8 |
| (C1) | 0.87 | 2.90 | >74 | 12 |
| (C3) | 1.58 | 3.48 | >40 | 8 |
| (C2) | 0.15 | 0.33 | >310 | 4 |
| Miltefosine^{a} | 3.50 ± 0.40 | | CC₅₀ 38.5 ± 4.18 µM | |

| | | | | |
|---|---|---|---|---|
| SI = selectivity index (CC₅₀/IC₅₀), n = number of independent measurements; "Data from IJP: Drugs and Drug Resistance, 2018, 8, 81-86. | | | | |

**Table 2. Results for compounds tested for L. donovani (MHOM/ET/67/L82) activity**

| Compound | IC₅₀ (µM) | IC₉₀ (µM) | SI | n |
|---|---|---|---|---|
| (C4) | 0.16 | 0.41 | >360 | 8 |
| (C1) | 1.39 | 2.32 | >63 | 16 |
| (C3) | 1.57 | 6.48 | >51 | 4 |
| (C2) | 0.14 | 0.65 | >580 | 4 |
| Miltefosine^{a} | 1.80 ± 0.40 | | CC₅₀ 38.5 ± 4.18 µM | |

| | | | | |
|---|---|---|---|---|
| SI = selectivity index (CC₅₀/IC₅₀), n = number of independent measurements; "Data from IJP: Drugs and Drug Resistance, 2018, 8, 81-86. | | | | |

### Comparative in vitro efficacy evaluation of selected DNDi lead compounds against a panel of visceral and cutaneous Leishmania species.

### Collection and culture of primary peritoneal macrophages

Female Swiss mice (BW ~20 g) were purchased from a commercial source (Janvier France) and kept in quarantine for 1 week. The animals were housed in group and drinking water was available *ad libitum.* To harvest peritoneal macrophages, the mice were first stimulated by intraperitoneal injection of 1 mL of starch solution (2% in PBS). After 24 h, macrophages were collected in RPMI-1640 supplemented with 5% iFCS, 2% penicillin-streptomycin and 1% L-glutamine by peritoneal lavage and immediately distributed in a 96-well plate at a concentration of 30,000 cells/well. The cells were incubated for 24 h at 37°C and 5 % CO2 to allow cell attachment.

### In vitro infection

Strains of *L. donovani, L. infantum, L. major, L. tropica, L. guyanensis* and *L. panamensis* were selected based on their intrinsic susceptibility to the commonly used reference drugs and their geographic distribution. All strains were cultivated in HOMEM promastigote medium and sub-cultured twice weekly. Passage numbers were kept as low as possible to avoid loss of virulence. For infection of the macrophages, metacyclic promastigote cultures were examined light microscopically for the abundant presence of metacyclic promastigotes. Upon counting, promastigotes were centrifuged and diluted in RPMI-1640 to the desired concentration. The infection ratio varied based on the ability of the strain to adequately infect the host cells and ranged from 5 to 20 promastigotes per macrophage. Macrophages were infected by adding 100 µL promastigote suspension per well onto the macrophages. When *ex vivo* amastigotes were available (only for two lab strains), macrophages were infected at a ratio of 10/1. Cells were incubated in presence of 5% CO2 at 37° for VL strains and at 34°C for CL strains.

### Drug-susceptibility assay

Stock solutions of the DND*i* lead compounds were prepared in 100% DMSO at 20 mM and robotically further diluted in water. Miltefosine (MIL) was included as reference and formulated in water at a concentration of 20 mM. The medium was discarded from the plates to remove any residual non-internalized promastigotes and replaced by fresh RPMI-1640 medium supplemented with iFBS, P/S and L-glutamine (as described above). Next, a 2-fold dilution series of each lead compound was added to the plate with 64 µM as the highest in test-concentration for the DNDi leads and 40 µM for MIL. The 96-well plates were further incubated at 34-37°C and 5% CO2. For staining, the culture medium was discarded, and the plates were left to dry for 2-4 hours. The cells were then fixated with methanol for 10 minutes and stained with a 1/5 Giemsa dilution in water for 15 minutes. The percentage infection-inhibition compared to a positive control (untreated infected macrophages) was microscopically determined and used to calculate the IC₅₀ and IC₉₀ values.

Results showing the anti-infective efficacy *in vitro* for compounds **C1, C3, C4,** and miltefosine against clinically isolated strains of *L. donovani, L. infantum, gabrielL. major, L. tropica, L. guyanensis, and L. panamensis* are shown in table 3.

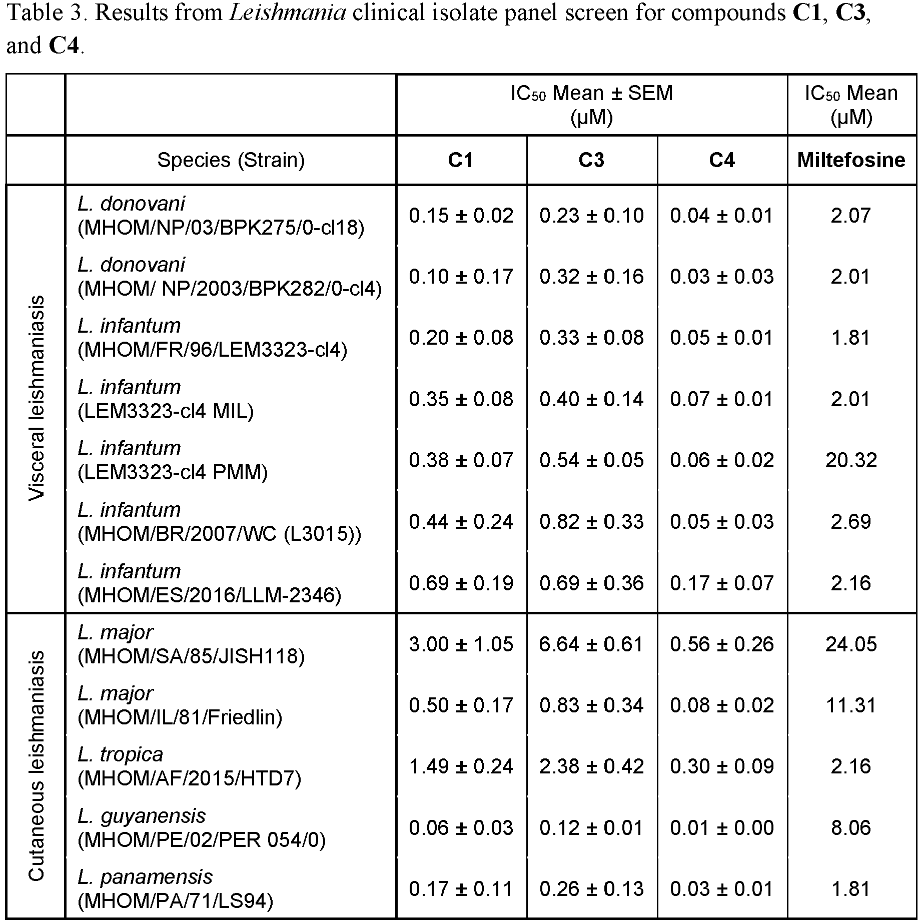

### In vivo testing

The *in vivo* efficacy of the compounds **C1, C3, C4,** and miltefosine was evaluated in a VL hamster model infected with *L. infantum.* The *in vivo* studies were performed in accordance with the methods described by Van den Kerkhof et al. [IJP: Drugs and Drug Resistance, 2018, 8, Issue 1, 81-86]. Following treatment, the reduction of parasite load vs. control was determined in different body compartments (liver, spleen, and bone marrow) with results presented in Table 4.

**Table 4. Hamster in vivo efficacy**

| | | | | ***% Parasite reduction*** | | |
|---|---|---|---|---|---|---|
| *Compound* | Dosing interval | Dose (mg/kg) | Duration (Days) | Liver | Spleen | Bone marrow |
| *(C1)* | bid | 50 | 5 | 99.2 | 95.3 | 97.4 |
| | bid | 25 | 5 | 95.9 | 95.2 | 98.9 |
| *(C3)* | qd | 25 | 5 | 98.4 | 99.0 | 86.7 |
| | qd | 12.5 | 5 | 96.1 | 98.4 | 89.9 |
| *(C4)* | qd | 50 | 5 | 99.7 | 99.9 | 99.9 |
| | qd | 25 | 5 | 99.6 | 99.9 | 99.4 |
| | bid | 12.5 | 5 | 99.7 | 99.9 | 99.6 |
| | bid | 6.25 | 5 | 96.9 | 97.6 | 93.6 |
| *Miltefosine* | qd | 40 | 5 | 97.0-98.2 | 95.7-99.5 | 92.1-99.0 |

## Claims

1. A compound of Formula (I) 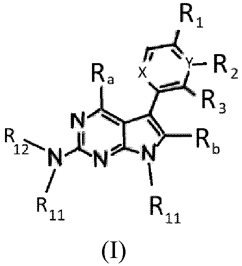 Wherein:
- each one of Rₐ and R_{b} denotes a group -CH₃ or -CF₃;
- Both X and Y denote a carbon atom or both X and Y denote a nitrogen atom when R₂ is absent;
- R₁ denotes a group selected from -OCH₃, -OCH₂CF₃, -CN, -CF₃, or halogen;
- R₂ is present when Y denotes a carbon atom, and denotes a hydrogen atom, or a group -OCH₃,
- R₃ denotes a group selected from -OCH₃, -CN, -CF₃ or halogen, or
- R₃ forms together with R₂ a group -OCH₂O- ;
- each one of R₁₁ and R₁₂ denotes a hydrogen atom;
or a pharmaceutically acceptable salt thereof.

2. Compound according to claim 1, wherein each one of Ra and Rb denotes a group -CH₃.

3. Compound according to claims 1 or 2, wherein R₁ is selected from -CN, halogen, - OCH₃ and -OCH₂CF₃.

4. Compound according to one of claims 1 to 3, the compound being on one of formulae (Ib) and (Ic) 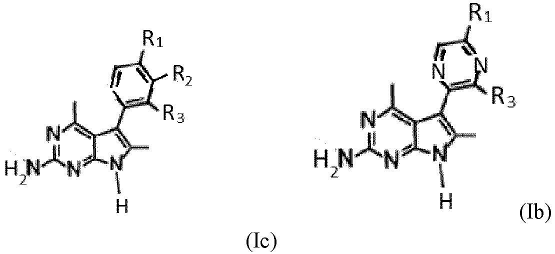 Wherein R₁, R₂ and R₃ are as defined in one of claims 1 to 3.

5. Compound according to one of claims 1 to 4, wherein the compound is selected from one of the following compounds C1, C2, C3 and C4
| **Compound** | **Formula** |
|---|---|
| **C1** | 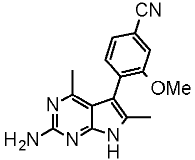 |
| 4-(2-amino-4,6-dimethyl-7H-pyrrolo[2,3-d]pyrimidin-5-yl)-3-methoxybenzonitrile | |
| **C2** | 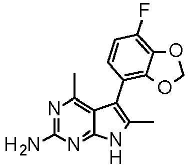 |
| 5-(7-fluoro-1,3-benzodioxol-4-yl)-4,6-dimethyl-7H-pyrrolo[2,3-d]pyrimidin-2-amine | |
| **C3** | 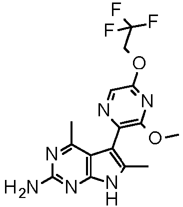 |
| 5-[3-methoxy-5-(2,2,2-trifluoroethoxy)pyrazin-2-yl]-4,6-dimethyl-7H-pyrrolo[2,3-d]pyrimidin-2-amine | |
| **C4** | 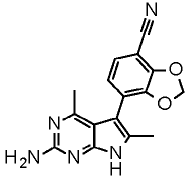 |
| 7-(2-amino-4,6-dimethyl-7H-pyrrolo[2,3-d]pyrimidin-5-yl)-1,3-benzodioxole-4-carbonitrile | |

6. Compound according to one of claims 1 to 5 for use as a medicament.

7. Compound according to one of claims 1 to 6 for use in the treatment of neglected tropical diseases.

8. Compound for use according to claim 7, wherein said neglected tropical disease is cutaneous leishmaniasis (CL), visceral leishmaniasis (VL), or mucocutaneous leishmaniasis (MCL).

9. Compound for use according to one of claims 6 to 8, said compound being used in combination with another known drugs selected from the groups consisting of meglumine antimoniate, sodium stibogluconate (SSG), liposomal amphotericin B, paromomycin sulfate, and miltefosine.

10. A process for the manufacture of a compound of formula (I), as defined in one of claims 1 to 5, comprising the step of reacting an intermediate of Formula (II) 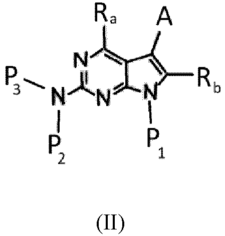
wherein Rₐ and R_{b} are as above described for formula (I), (Ia) or the related embodiments and wherein :
- A denotes a group selected from I, Br, or a group dioxaborolan-2-yl;
- P₁, P₂ and P₃ independently denote an hydrogen atom or a protective group selected from a terbutoxycarbonyl (Boc), 2-(trimethylsilyl)ethoxymethyl (SEM), a paramethoxybenzyl (PMB); or one of the groups R₁₁ and R₁₂;
with an intermediate of Formula (III) 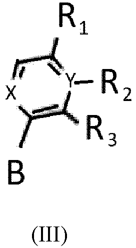
wherein X, Y, R₁, R₂ and R₃ are as above-defined for formulae (I), (Ia) and related embodiments and wherein B denotes :
- a group dioxaborolane-2-yl when A denotes I or Br,
- or I or Br when A denotes dioxaborolane-2-yl;
in the presence of a Palladium based catalyst and a carbonate or a phosphate, at a temperature comprised between 60°C and 150°C, in an aqueous solvent comprising dioxane or DMSO or a mixture thereof, so that a pyrrolo[2,3-d]pyrimidin-2-amine derivative of Formula (IV) is obtained.

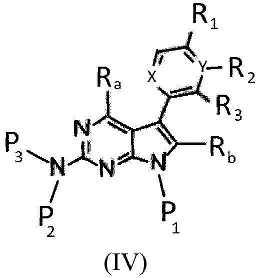

11. Process according to claim 10, further comprising a step of reacting said pyrrolo[2,3-d]pyrimidin-2-amine derivative of Formula (IV) with suitable derivatives under suitable reaction conditions so that a compound as defined in one the claims 1 to 6 is obtained.

12. A pharmaceutical composition comprising a compound as defined in one of claims 1 to 5 or a pharmaceutically acceptable salt thereof, and at least one of pharmaceutically acceptable additive, buffer, excipient or carrier.

13. Kit comprising a compound of formula (I) as defined in claim 1, and instructions related to the medical use thereof.

## Patentansprüche

1. Eine Verbindung der Formel (I) 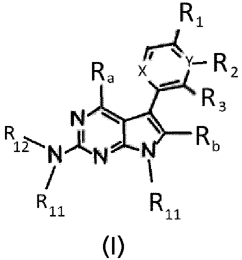 worin:
- jedes von Rₐ und R_{b} eine Gruppe -CH₃ or -CF₃ bezeichnet;
- sowohl X als auch Y ein Kohlenstoffatom oder sowohl X als auch Y ein Stickstoffatom bezeichnen, wenn R₂ fehlt;
- R₁ eine Gruppe ausgewählt aus -OCH₃, -OCH₂CF₃, -CN, -CF₃, oder Halogen bezeichnet;
- R₂ vorhanden ist, wenn Y ein Kohlenstoffatom bezeichnet, und ein Wasserstoffatom oder eine Gruppe -OCH₃ bezeichnet,
- R₃ eine Gruppe ausgewählt aus -OCH₃, -CN, -CF₃ oder Halogen bezeichnet, oder
- R₃ zusammen mit R₂ eine Gruppe -OCH₂O- bildet;
- R₁₁ und R₁₂ jeweils ein Wasserstoffatom bezeichnen;
oder ein pharmazeutisch verträgliches Salz davon.

2. Verbindung gemäss Anspruch 1, worin jedes von Rₐ und R_{b} jeweils eine Gruppe -CH₃ bezeichnen.

3. Verbindung gemäss Ansprüchen 1 oder 2, worin R₁ aus -CN, Halogen, - OCH₃ und -OCH₂CF₃ ausgewählt ist.

4. Verbindung gemäss einem der Ansprüche 1 bis 3, wobei die Verbindung eine der Formeln (Ib) und (Ic) ist: 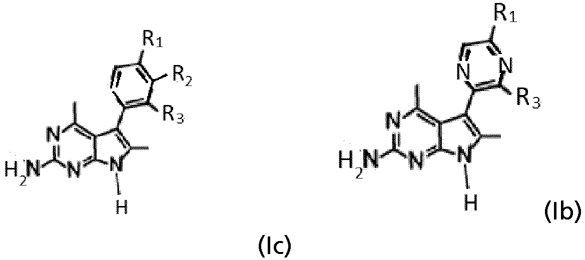 worin R₁, R₂ und R₃ wie in einem der Ansprüche 1 bis 3 definiert sind.

5. Verbindung gemäss einem der Ansprüche 1 bis 4, worin die Verbindung aus einer der folgenden Verbindungen Cl, C2, C3 und C4 ausgewählt ist:
| **Verbindung** | **Formel** |
|---|---|
| **C1** | 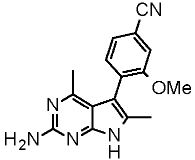 |
| 4-(2-Amino-4,6-dimethyl-7H-pyrrolo[2,3-d]pyrimidin-5-yl)-3-Methoxybenzonitril | |
| **C2** | 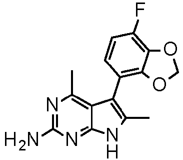 |
| 5-(7-Fluoro-1,3-benzodioxol-4-yl)-4,6-dimethyl-7H-pyrrolo[2,3-d]pyrimidin-2-Amin | |
| **C3** | 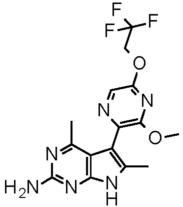 |
| 5-[3-Methoxy-5-(2,2,2-trifluoroethoxy)pyrazin-2-yl]-4,6-dimethyl-7H-pyrrolo[2,3-d]pyrimidin-2-Amin | |
| **C4** | 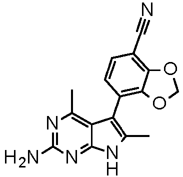 |
| 7-(2-Amino-4,6-dimethyl-7H-pyrrolo[2,3-d]pyrimidi n-5-yl)-1,3-benzodioxole-4-Carbonitril | |

6. Verbindung gemäss einem der Ansprüche 1 bis 5 zur Verwendung als Arzneimittel.

7. Verbindung gemäss einem der Ansprüche 1 bis 6 zur Verwendung bei der Behandlung vernachlässigter Tropenkrankheiten.

8. Verbindung gemäss Anspruch 7, worin es sich bei der besagten vernachlässigten Tropenkrankheit um kutane Leishmaniose (CL), viszerale Leishmaniose (VL) oder mukokutane Leishmaniose (MCL) handelt.

9. Verbindung zur Verwendung gemäss einem der Ansprüche 6 bis 8, wobei die besagte Verbindung in Kombination mit anderen bekannten Arzneimitteln verwendet wird, ausgewählt aus den Gruppen bestehend aus Megluminantimoniat, Natriumstibogluconat (SSG), liposomalem Amphotericin B, Paromomycinsulfat und Miltefosin.

10. Verfahren zur Herstellung einer Verbindung der wie in einem der Ansprüche 1 bis 5 definierten Formel (I), umfassend den Schritt des Reagierens eines Zwischenprodukts der Formel (II) 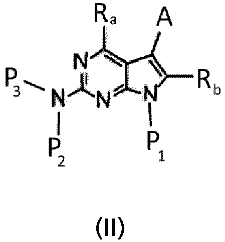
worin Rₐ und R_{b} wie oben für Formel (I), (la) oder die zugehörigen Ausführungsformen beschrieben sind, und worin:
- A eine Gruppe ausgewählt aus I, Br oder einer Dioxaborolan-2-yl-Gruppe bezeichnet;
- P₁, P₂ und P₃ unabhängig voneinander bezeichnen: ein Wasserstoffatom oder eine Schutzgruppe, ausgewählt aus Terbutoxycarbonyl (Boc), 2-(Trimethylsilyl)ethoxymethyl (SEM), Paramethoxybenzyl (PMB); oder eine der Gruppen R₁₁ und R₁₂;
mit einem Zwischenprodukt der Formel (III) 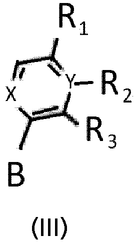
worin X, Y, R₁, R₂ und R₃ wie oben für die Formeln (I), (la) und verwandte Ausführungsformen definiert sind, und worin B bezeichnet:
- eine Dioxaborolan-2-yl-Gruppe, wenn A I oder Br bezeichnet,
- oder I oder Br, wenn A Dioxaborolan-2-yl bezeichnet;
in Gegenwart eines auf Palladium basierenden Katalysators und eines Carbonats oder Phosphats bei einer Temperatur zwischen 60°C und 150°C in einem wässrigen Lösungsmittel, welches Dioxan oder DMSO oder eine Mischung davon enthält, so dass ein Pyrrolo[2,3-d]pyrimidin-2-amin-Derivat der Formel (IV) erhalten wird:

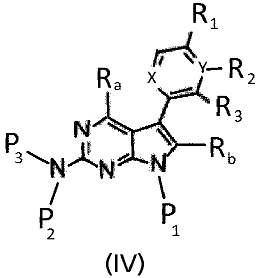

11. Verfahren gemäss Anspruch 11, welches ausserdem einen Schritt des Reagierens des Pyrrolo[2,3-d]pyrimidin-2-amin-Derivats der Formel (IV) mit geeigneten Derivaten unter geeigneten Reaktionsbedingungen umfasst, so dass eine Verbindung gemäss der Definition in einem der Ansprüche 1 bis 6 erhalten wird.

12. Pharmazeutische Zusammensetzung, welche eine Verbindung gemäss einem der Ansprüche 1 bis 5 oder ein pharmazeutisch verträgliches Salz davon und mindestens einen pharmazeutisch verträglichen Zusatzstoff, Puffer, Hilfsstoff oder Träger umfasst.

13. Kit, umfassend eine Verbindung der wie in Anspruch 1 definierten Formel (I), sowie Anweisungen für deren medizinische Verwendung.

## Revendications

1. Un composé de Formule (I) 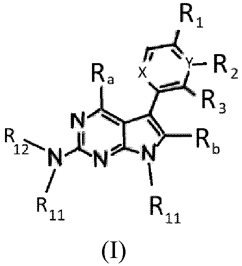 dans lequel:
- Chacun des groupes Rₐ et R_{b} désigne un groupe -CH₃ ou -CF₃;
- Les deux groupes X et Y désignent un atome de carbone ou les deux groupes X et Y désignent un atome d'azote si R₂ est absent;
- R₁ désigne un groupe sélectionné parmi -OCH₃, -OCH₂CF₃, -CN, -CF₃, ou un halogène;
- R₂ est présent si Y désigne un atome de carbone, et désigne un atome d'hydrogène, ou un groupe -OCH₃,
- R₃ désigne un groupe sélectionné parmi -OCH₃, -CN, -CF₃ ou un halogène, ou
- R₃ forme avec le groupe R₂ un groupe -OCH₂O- ;
- Chacun des groupes R₁₁ et R₁₂ désigne un atome d'hydrogène,
ou un de ses sels pharmaceutiques acceptables.

2. Composé selon la revendication 1, dans lequel chacun des groupes Ra et Rb désigne un groupe -CH₃.

3. Composé selon l'une des revendications 1 ou 2, dans lequel le groupe R₁ est sélectionné parmi -CN, un halogène, -OCH₃ et -OCH₂CF₃.

4. Composé selon l'une des revendications 1 à 3, le composé étant de l'une des formules (Ib) et (Ic) 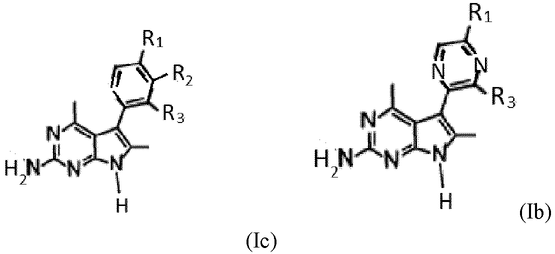 où les groupes R₁, R₂ et R₃ sont tels que définis dans une des revendications 1 à 3.

5. Composé selon l'une des revendications 1 à 4, dans lequel le composé est sélectionné parmi l'un des composés C1, C2, C3 et C4 suivants
| **Composé** | **Formule** |
|---|---|
| **C1** | 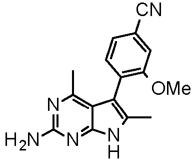 |
| 4-(2-amino-4,6-dimethyl-7H-pyrrolo[2,3-d]pyrimidin-5-yl)-3-methoxybenzonitrile | |
| **C2** | 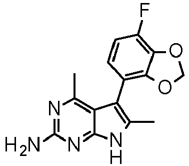 |
| 5-(7-fluoro-1,3-benzodioxol-4-yl)-4,6-dimethyl-7H-pyrrolo[2,3-d]pyrimidin-2-amine | |
| **C3** | 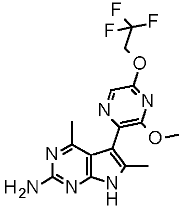 |
| 5-[3-methoxy-5-(2,2,2-trifluoroethoxy)pyrazin-2-yl]-4,6-dimethyl-7H-pyrrolo[2,3-d]pyrimidin-2-amine | |
| **C4** | 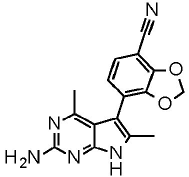 |
| 7-(2-amino-4,6-dimethyl-7H-pyrrolo[2,3-d]pyrimidin-5-yl)-1,3-benzodioxole-4-carbonitrile | |

6. Composé selon l'une des revendications 1 à 5 pour être utilisé comme un médicament.

7. Composé selon l'une des revendications 1 à 6 pour être utilisé dans le traitement des maladies tropicales négligées.

8. Composé pour être utilisé selon la revendication 7, où ladite maladie tropicale négligée est la leishmaniose cutanée (LC), la leishmaniose viscérale (LV), ou la leishmaniose muco-cutanée (LMC).

9. Composé pour être utilisé selon l'une des revendications 6 à 8, ledit composé étant utilisé en combinaison avec d'autres médicaments connus sélectionnés parmi le groupe consistant en l'antimoniate de meglumine, le stibogluconate de sodium (SSG), l'amphotéricine B liposomale, le sulfate de paromomycine, et le miltefosine.

10. Un procédé pour la production d'un composé de formule (I), tel que défini dans l'une des revendications 1 to 5, comprenant les étapes de faire réagir un intermédiaire de Formule (II) 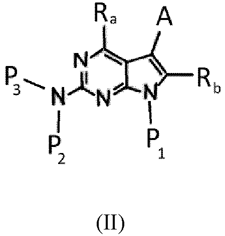
où Rₐ et R_{b} sont tels que définis plus haut pour la formule (I), (Ia) ou les modes de réalisation correspondants et où :
- A désigne un groupe sélectionné parmi I, Br, ou un groupe dioxaborolan-2-yl;
- P₁, P₂ et P₃ désignent indépendamment un atome d'hydrogène ou un groupe protecteur sélectionné parmi un terbutoxycarbonyl (Boc), éthoxyméthyl-2-(trimethylsilyl) (SEM), un paramethoxybenzyl (PMB); ou l'un des groupes R₁₁ et R12;
Avec un intermédiaire de Formule (III) 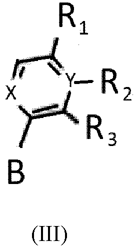
où X, Y, R₁, R₂ and R₃ sont tels que définis ci-dessus pour la formule (I), (Ia) et les modes de réalisation correspondants et où B désigne :
- un group dioxaborolane-2-yl si A désigne I ou Br,
- ou I ou Br si A désigne le dioxaborolane-2-yl;
en présence d'un catalyseur à base de palladium et un carbonate ou un phosphate, à une température comprise entre 60°C et 150°C, dans un solvant aqueux comprenant du dioxane ou du DMSO ou un de leurs mélanges, de sorte qu'un dérivé d'amine-2-pyrrolo[2,3-d]pyrimidine de Formule (IV) soit obtenu

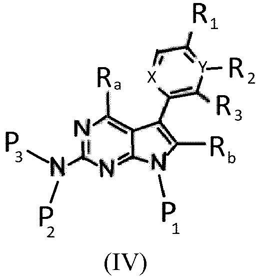

11. Procédé selon la revendication 10, comprenant en outre une étape de faire réagir ledit dérivé d'amine-2-pyrrolo[2,3-d]pyrimidine de Formule (IV) avec des dérivés adéquats dans les conditions adéquates de sorte qu'un composé tel que défini dans l'une des revendications 1 à 6 soit obtenu.

12. Une composition pharmaceutique comprenant un composé tel que défini dans l'une des revendications 1 à 5 ou un de ses sels pharmaceutiques acceptables, et au moins un additif, tampon, excipient ou vecteur pharmaceutique acceptable.

13. Kit comprenant un composé de formule (I) tel que défini dans la revendication 1, et des instructions relatives à son usage médical.
